(19) **Europäisches Patentamt / European Patent Office / Office européen des brevets**

(11) **EP 4 144 760 A1**

(12) **EUROPEAN PATENT APPLICATION**
published in accordance with Art. 153(4) EPC

(43) Date of publication:
**08.03.2023 Bulletin 2023/10**

(21) Application number: **21797169.6**

(22) Date of filing: **29.04.2021**

(51) International Patent Classification (IPC):
*C07K 16/28* (2006.01)   *C12N 15/13* (2006.01)
*C12N 15/63* (2006.01)   *C12N 5/16* (2006.01)
*A61K 39/395* (2006.01)   *A61P 35/00* (2006.01)

(52) Cooperative Patent Classification (CPC):
**A61K 39/395; A61P 35/00; C07K 16/00;
C07K 16/28; C12N 5/16; C12N 15/63**

(86) International application number:
**PCT/CN2021/090794**

(87) International publication number:
**WO 2021/219046 (04.11.2021 Gazette 2021/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.04.2020   CN 202010365705**

(71) Applicant: **Chia Tai Tianqing Pharmaceutical
Group Co., Ltd.
Lianyungang City, Jiangsu 222062 (CN)**

(72) Inventors:
• **ZHANG, Bing
  Nanjing City, Jiangsu 211100 (CN)**
• **ZHAO, Wei
  Nanjing City, Jiangsu 211100 (CN)**
• **DU, Min
  Nanjing City, Jiangsu 211100 (CN)**

• **LU, Yamin
  Nanjing City, Jiangsu 211100 (CN)**
• **MA, Yimin
  Nanjing City, Jiangsu 211100 (CN)**
• **DU, Xiuzhen
  Nanjing City, Jiangsu 211100 (CN)**
• **LU, Na
  Nanjing City, Jiangsu 211100 (CN)**
• **MA, Zhaoxiong
  Nanjing City, Jiangsu 211100 (CN)**
• **ZHANG, Xiquan
  Nanjing City, Jiangsu 211100 (CN)**

(74) Representative: **Müller Schupfner & Partner
Patent- und Rechtsanwaltspartnerschaft mbB
Bavariaring 11
80336 München (DE)**

Remarks:
The complete document including Reference
Table(s) and the Sequence Listing(s) can be
downloaded from the EPO website

(54) **ANTIGEN BINDING CONSTRUCTS TARGETING HER2 AND USES THEREOF**

(57)    Disclosed are antigen binding constructs targeting HER2 and uses thereof. Specifically provided are antigen binding constructs, a nucleic acid that encodes same, a vector that comprises said nucleic acid, a cell that comprises said vector, and a pharmaceutical composition that comprises the foregoing. Further provided are uses thereof in aspects such as treating subjects who suffer from HER2-expressing tumors, killing HER2-expressing tumor cells, or inhibiting the growth of HER2-expressing tumor cells.

EP 4 144 760 A1

**Description**

**TECHNICAL FIELD**

The present disclosure relates to an antigen-binding construct, and in particular to an HER2-targeted antigen-binding construct and use thereof.

**BACKGROUND**

Her2 (human epidermal growth factor receptor 2), also known as ErbB-2 (receptor tyrosine-protein kinase erbB-2), is a member of the human epidermal growth factor receptor (HER/EGFR/ERBB) family, which includes EGFR (ErbB-1), Her2/c-neu (ErbB-2), Her3 (ErbB-3) and Her4 (ErbB-4). Her2 protein contains an extracellular ligand binding domain (domains I-IV), a transmembrane domain, and an intracellular domain. The ligand of Her2 is unknown. Her2 and any of the other three receptors (ErbB-1, ErbB-3 and ErbB-4) form a heterodimer. The dimerization leads to the autophosphorylation of tyrosine residues in the cytoplasmic domain of the receptor and the activation of a variety of signaling pathways, including MAPK (mitogen-activated protein kinase), PI3K/Akt (phosphoinositide 3-kinase), PKC (protein kinase C), and STAT (signal transducer and activator of transcription). The expansion or overexpression of the Her2 gene plays an important role in the development and progression of some types of invasive breast cancer. 15%-30% of breast cancer patients are Her2 positive. Her2 is an important biomarker and a target of therapy for breast cancer patients. In addition, Her2 is found overexpressed in 7%-34% of gastric cancer patients and 30% of salivary duct carcinoma patients.

Several Her2-targeted antibody drugs for treating Her2-positive tumors, including Roche's monoclonal antibody drug Herceptin® that targets Her2 extracellular domain IV (ECD4), Roche's Perjeta® that targets Her2 extracellular domain II (ECD2), and Roche's ADC T-DM1® (ado-trastuzumab memtansine). The mechanisms of action of these monoclonal antibody drugs includes: 1) down-regulating Her2 signaling; 2) binding to Her2 to form epitopes of signaling heterodimers; 3) endocytosing Her2; and 4) ADCC (antibody dependent cellular cytotoxicity) of monoclonal antibodies. Monospecific targeting is not directed against other target epitopes that may be involved in signaling and pathogenesis, allowing for drug resistance and escape mechanisms.

Antibody aggregation can present difficulties in aspects such as expression and/or purification, immunogenicity, toxicity, degradation, impaired avidity, or loss of activity after storage. Therefore, in industrial applications, it is desirable to reduce antibody aggregation. Being single chain molecules, scFvs aggregate more easily, and may aggregate to form a dimer (diabody), a trimer (triabody) and a tetramer (tetrabody).

**SUMMARY**

The present disclosure provides an antigen-binding construct with reduced aggregation. The present disclosure further provides use of the antigen-binding construct.

In one aspect, the present disclosure provides an antigen-binding construct, which comprises a first antigen-binding fragment that is monovalent and specifically binds to ECD4 antigen of HER2 on an HER2-expressing cell, wherein the first antigen-binding fragment is an scFv; a heavy chain variable region of the first antigen-binding fragment comprises an amino acid mutation at position 30 according to the Kabat numbering system, and preferably, the amino acid is mutated into E; or/and a light chain variable region of the first antigen-binding fragment comprises an amino acid mutation at position 53 according to the Kabat numbering system, and preferably, the amino acid is mutated into Y

Alternatively, the present disclosure provides an antigen-binding construct, which comprises a first antigen-binding fragment that is monovalent and specifically binds to ECD4 antigen of HER2 on an HER2-expressing cell, wherein the first antigen-binding fragment is an scFv; a heavy chain variable region of the first antigen-binding fragment comprises an amino acid K or E at position 30 according to the Kabat numbering system, or/and a light chain variable region of the first antigen-binding fragment comprises an amino acid F or Y at position 53 according to the Kabat numbering system; however, when the heavy chain variable region of the first antigen-binding fragment comprises an amino acid K at position 30 according to the Kabat numbering system, the light chain variable region of the first antigen-binding fragment does not comprise an amino acid F at position 53 according to the Kabat numbering system.

In some embodiments, the heavy chain variable region of the first antigen-binding fragment comprises a K30E mutation according to the Kabat numbering system, or/and the light chain variable region of the first antigen-binding fragment comprises an F53Y mutation according to the Kabat numbering system. In some specific embodiments, the heavy chain variable region of the first antigen-binding fragment comprises a K30E mutation according to the Kabat numbering system. In some other specific embodiments, the light chain variable region of the first antigen-binding fragment comprises an F53Y mutation according to the Kabat numbering system. In some embodiments, the heavy chain variable region of the first antigen-binding fragment comprises a K30E mutation according to the Kabat numbering system, and the light chain variable region of the first antigen-binding fragment comprises an F53Y mutation according to the Kabat numbering system.

In some embodiments, the first antigen-binding fragment comprises a group of CDRs selected from:

i. a group comprising a heavy chain CDR1, a heavy chain CDR2 and a heavy chain CDR3, wherein the heavy chain CDR1, the heavy chain CDR2 and the heavy chain CDR3 comprise the amino acid sequences according to SEQ ID NOs: 27, 28 and 29, respectively;

ii. a group comprising a heavy chain CDR1, a heavy chain CDR2 and a heavy chain CDR3, wherein the heavy chain CDR1, the heavy chain CDR2 and the heavy chain CDR3 comprise amino acid sequences having at least 80% identity to the amino acid sequences according to SEQ ID NOs: 27, 28 and 29, respectively;

iii. a group comprising a light chain CDR1, a light chain CDR2 and a light chain CDR3, wherein the light chain CDR1, the light chain CDR2 and the light chain CDR3 comprise the amino acid sequences according to SEQ ID NOs: 30, 34 and 32, respectively;

iv. a group comprising a light chain CDR1, a light chain CDR2 and a light chain CDR3, wherein the light chain CDR1, the light chain CDR2 and the light chain CDR3 comprise amino acid sequences having at least 80% identity to the amino acid sequences according to SEQ ID NOs: 30, 34 and 32, respectively;

v. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 27, 28 and 29, respectively; and the light chain CDR1, the light chain CDR2 and the light chain CDR3 comprise the amino acid sequences according to SEQ ID NOs: 30, 34 and 32, respectively;

vi. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise amino acid sequences having at least 80% identity to the amino acid sequences according to SEQ ID NOs: 27, 28 and 29, respectively; and the light chain CDR1, the light chain CDR2 and the light chain CDR3 comprise amino acid sequences having at least 80% identity to the amino acid sequences according to SEQ ID NOs: 30, 34 and 32, respectively;

vii. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 43, 28 and 29, respectively; and the light chain CDR1, the light chain CDR2 and the light chain CDR3 comprise the amino acid sequences according to SEQ ID NOs: 30, 31 and 32, respectively;

viii. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 43, 28 and 29, respectively; and the light chain CDR1, the light chain CDR2 and the light chain CDR3 comprise the amino acid sequences according to SEQ ID NOs: 30, 33 and 32, respectively; and

ix. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 44, 28 and 29, respectively; and the light chain CDR1, the light chain CDR2 and the light chain CDR3 comprise the amino acid sequences according to SEQ ID NOs: 30, 31 and 32, respectively.

Wherein the sequence set forth in SEQ ID NO: 27 is GFNIX$_2$DTYIH, where X$_2$ is K or E; the sequence set forth in SEQ ID NO: 34 is SASX$_1$LYS, where X$_1$ is F or Y

In some embodiments, the first antigen-binding fragment comprises a heavy chain variable region of trastuzumab or a mutant thereof, and a light chain variable region of trastuzumab or a mutant thereof, wherein the mutant of the heavy chain variable region comprises a K30E mutation according to the Kabat numbering system, and the mutant of the light chain variable region comprises an F53Y mutation according to the Kabat numbering system.

In some embodiments, the first antigen-binding fragment is selected from:

i. the first antigen-binding fragment comprising a heavy chain variable region and a light chain variable region, which comprise the amino acid sequences according to SEQ ID NOs: 41 and 42, respectively;

ii. the first antigen-binding fragment comprising a heavy chain variable region and a light chain variable region, which comprise amino acid sequences having at least 80% identity to the amino acid sequences according to SEQ ID NOs: 41 and 42, respectively;

iii. the first antigen-binding fragment comprising a heavy chain variable region and a light chain variable region, which comprise the amino acid sequences according to SEQ ID NOs: 35 and 36, respectively;

iv. the first antigen-binding fragment comprising a heavy chain variable region and a light chain variable region, which comprise the amino acid sequences according to SEQ ID NOs: 35 and 39, respectively; and

v. the first antigen-binding fragment comprising a heavy chain variable region and a light chain variable region, which comprise the amino acid sequences according to SEQ ID NOs: 40 and 36, respectively;

wherein the sequence set forth in SEQ ID NO: 41 is:
EVQLVESGGGLVQPGGSLRLSCAASGFNIX$_2$DTYIHWVRQAPGKGLEWVARIYPTNGYTRYADSVKGRFT
ISADTSKNTAYLQMNSLRAEDTAVYYCSRWGGDGFYAMDYWGQGTLUTVSS, where X$_2$ is K or E;
the sequence set forth in SEQ ID NO: 42 is:
DIQMTQSPSSLSASVGDRVTITCRASQDVNTAVAWYQQKPGKAPKLLIYSASX$_1$LYSGVPSRFSGSRSGTDF
TLTISSLQPEDFATYYCQQHYTTPPTFGQGTKVEIK, where X$_1$ is F or Y

In some embodiments, the antigen-binding construct described herein further comprises a second antigen-binding fragment that is monovalent and specifically binds to ECD2 antigen of HER2 on an HER2-expressing cell. In some embodiments, the second antigen-binding fragment is an Fab. In some embodiments, the second antigen-binding fragment comprises a heavy chain CDR1, a heavy chain CDR2, a heavy chain CDR3, a light chain CDR1, a light chain CDR2 and a light chain CDR3 of pertuzumab. In some embodiments, the second antigen-binding fragment comprises a heavy chain variable region of pertuzumab and a light chain variable region of pertuzumab.

In one aspect, the present disclosure provides a pharmaceutical composition, which comprises the antigen-binding construct described herein and a pharmaceutically acceptable carrier.

In one aspect, the present disclosure provides an isolated nucleic acid or a collection of isolated nucleic acids, which comprises at least one nucleic acid sequence encoding at least one antigen-binding fragment of the antigen-binding construct described herein.

In one aspect, the present disclosure provides a vector or a collection of vectors, which comprises one or more of the nucleic acid and the collection of nucleic acids described herein.

In one aspect, the present disclosure provides an isolated cell, which comprises the nucleic acid or the collection of nucleic acids described herein, or the vector or the collection of vectors described herein.

In one aspect, the present disclosure provides a method for preparing the antigen-binding construct described herein, which comprises: culturing a host cell under conditions suitable for expression of the antigen-binding construct, wherein the host cell comprises a nucleic acid encoding the antigen-binding construct described herein, or the vector or the collection of vectors described herein; and purifying the construct.

In another aspect, the present disclosure provides a method for treating a subject having an HER2-expressing tumor, which comprises administering to the subject a therapeutically effective amount of the antigen-binding construct described herein or the pharmaceutical composition described herein.

In another aspect, the present disclosure provides a method for killing an HER2-expressing tumor cell or inhibiting growth of an HER2-expressing tumor cell, which comprises contacting the tumor cell with the antigen-binding construct described herein.

In another aspect, the present disclosure provides a method for inhibiting, reducing or blocking HER2 signaling in a cell, which comprises administering to the cell an effective amount of the antigen-binding construct described herein or the pharmaceutical composition described herein.

In another aspect, the present disclosure provides a method for detecting or measuring HER2 in a sample, which comprises contacting the sample with the antigen-binding construct described herein and detecting or measuring a binding complex.

In yet another aspect, the present disclosure provides a method for enhancing resistance of an antigen-binding construct to aggregation, wherein the antigen-binding construct comprises a first antigen-binding fragment that is monovalent and specifically binds to ECD4 antigen of HER2 on an HER2-expressing cell, the first antigen-binding fragment being an scFv; the method comprises modifying the antigen-binding construct so that a heavy chain variable region of the first antigen-binding fragment comprises an amino acid mutation at position 30 according to the Kabat numbering system and the amino acid is mutated into E, or/and that a light chain variable region of the first antigen-binding fragment comprises an amino acid mutation at position 53 according to the Kabat numbering system and the amino acid is mutated into Y

## BRIEF DESCRIPTON OF THE DRAWINGS

FIG. 1 shows the glycan mapping analysis of an anti-HER2 bispecific antibody Expi Her2-2;

FIG. 2 shows the glycan mapping analysis of an anti-HER2 bispecific antibody 23C2 Her2-2;

FIG. 3 shows the killing rates of the combination of trastuzumab and pertuzumab and anti-HER2 bispecific antibodies against BT474 tumor cells;

FIG. 4 shows the killing rates of trastuzumab, the combination of trastuzumab and pertuzumab, T-DM1 and anti-HER2 bispecific antibodies against NCI-N87 tumor cells;

FIG. 5 shows the killing rates of trastuzumab, the combination of trastuzumab and pertuzumab, T-DM1 and anti-HER2 bispecific antibodies against JIMT-1 tumor cells;

FIG. 6 shows the inhibition of proliferation of BT474 tumor cells by trastuzumab, the combination of trastuzumab and pertuzumab and an anti-HER2 bispecific antibody;

FIG. 7 shows the effects of the combination of trastuzumab and pertuzumab and anti-HER2 bispecific antibodies on changes in mouse tumor volume in a gastric cancer N87 mouse xenograft tumor model;

FIG. 8 shows the effects of the combination of trastuzumab and pertuzumab and anti-HER2 bispecific antibodies on changes in mouse body weight in the gastric cancer N87 mouse xenograft tumor efficacy; and

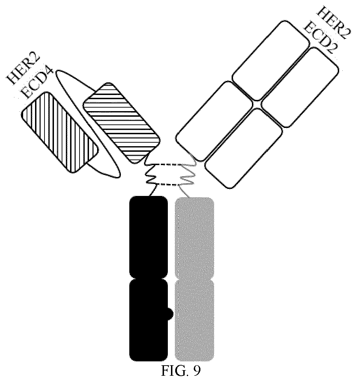

FIG. 9 shows a structure of some exemplary anti-HER2 bispecific antibodies, wherein a dimeric Fc is depicted with one chain shown in black (a first Fc polypeptide) and another chain shown in gray (a second Fc polypeptide); a first antigen-binding fragment is an scFv, fused with the first Fc polypeptide, and a second antigen-binding fragment is an Fab, fused with the second Fc polypeptide.

## DETAILED DESCRIPTION

### Terminology

The term "antigen-binding construct" refers to any agent that is capable of binding to an antigen, such as polypeptides or polypeptide complexes. In some aspects, the antigen-binding construct is a polypeptide that specifically binds to an antigen of interest. The antigen-binding construct may be a monomer, a dimer, a polymer, a protein, a peptide, or a protein or peptide complex; an antibody or an antigen-binding portion thereof; an scFv, etc. The antigen-binding construct may be a monospecific, bispecific or multispecific polypeptide construct. In some aspects, the antigen-binding construct may include, for example, one or more antigen-binding components (e.g., Fab or scFv) linked to one or more Fc. Other examples of the antigen-binding construct are described below and provided in the examples.

The term "bispecific" is intended to indicate that any agent, such as an antigen-binding construct, comprises two antigen-binding portions (e.g., antigen-binding fragments), each of which has unique binding specificity. For example, a first antigen-binding fragment binds to an epitope on a first antigen, while a second antigen-binding fragment binds to an epitope on a second antigen. As another example, a first antigen-binding fragment and a second antigen-binding fragment each bind to a different epitope of the same antigen.

"Antibody" is used in its broadest sense and specifically encompasses intact monoclonal antibodies, polyclonal antibodies, multispecific antibodies (e.g., bispecific antibodies), multifunctional antibodies and antibody fragments so long as they possess the desired biological activity.

The term "variable region" refers to a domain of about 100 to 110 or more amino acids defined by the N-terminal domain of the light or heavy chain of an antibody that is primarily responsible for antigen recognition. The terms light chain variable region (VL) and heavy chain variable region (VH) refer to these light and heavy chain domains, respectively.

The term "CDR" (complementarity-determining region), also known as "hypervariable region", refers to each region of an antibody variable domain which is highly variable in sequence and/or forms a structurally defined loop. Natural four-chain antibodies typically comprise six CDRs, three in the heavy chain variable region and three in the light chain variable region.

The term "Fab" means comprising the constant domain (CL) of the light chain and the first constant domain (CH1) of the heavy chain, together with the variable domains VL (light chain variable region) and VH (heavy chain variable region) in the light chain and heavy chain, respectively. The variable domain comprises complementarity-determining regions (CDRs) that are involved in antigen-binding.

The term "scFv" includes the VH and VL domains of an antibody, wherein these domains are present in a single polypeptide chain. In some embodiments, scFv further comprises a polypeptide linker between the VH and VL domains that enables the scFv to form the required structure for antigen-binding.

The term "ECD" refers to an extracellular domain. The extracellular domain of HER2 includes four domains, which are ECD1, ECD2, ECD3 and ECD4.

Generic term "trastuzumab" refers to a recombinant humanized monoclonal antibody that selectively acts on ECD4 of human epidermal growth factor receptor-2 (HER2) and can be used to treat HER2-positive cancer, an example of which is the commercially available therapeutic monoclonal antibody product under the trade name HERCEPTIN®.

Generic term "pertuzumab" refers to a recombinant humanized monoclonal antibody that selectively acts on ECD2 of human epidermal growth factor receptor-2 (HER2) and can be used to treat HER2-positive cancer.

The term "HER2" refers to the second member of the EGFR family, which has tyrosine kinase activity.

The term "operably linked" refers to a functional linkage between two or more peptide or polypeptide domain or nucleic acid (e.g., DNA) segments. In the context of proteins, antibodies or other polypeptides, the term "operably linked" means that two or more amino acid segments are linked so as to produce a functional polypeptide.

The term "$EC_{50}$" refers to the effective concentration that induces 50% of the maximal response of an antigen-binding construct. As used herein, the term "$IC_{50}$" refers to the inhibitory concentration that induces 50% of the maximal response of an antigen-binding construct. Both $EC_{50}$ and $IC_{50}$ can be measured by ELISA or FACS analysis or any other method known in the art.

The term "KD" as used herein refers to the equilibrium dissociation constant, expressed in molar concentration (M). The KD value of an antigen-binding construct can be determined using methods well known in the art. One preferred method of determining the KD value of an antigen-binding construct is to use surface plasmon resonance, more preferably to use a biosensor system, such as a Biacore system.

The term "treatment" refers to a measure taken to statistically significantly treat, cure, alleviate, relieve, alter, remedy, ameliorate, improve or affect an illness (e.g., a disease) and a symptom of the illness, or prevent or delay the onset of the symptom, a complication and biochemical indicators, or otherwise prevent or inhibit the further progression of the disease, illness or disorder.

The term "therapeutically effective amount" refers to the amount of an antigen-binding construct or a composition necessary to provide a therapeutic and/or prophylactic benefit to a subject.

The term "subject" includes any human or non-human animal. The term "non-human animal" includes all vertebrates, e.g., mammals and non-mammals, such as non-human primates, sheep, dog, cat, horse, cow, chicken, amphibians, and reptiles. Preferably, the subject according to the present disclosure is a human. The terms "patient" and "subject" are used interchangeably unless otherwise indicated.

As used herein, "about" means being within an acceptable error range determined by those of ordinary skill in the art for a particular value, which will depend in part on how the value is measured or determined, i.e., the limitation by the measurement system. For example, "about" may mean being within 1 or more than 1 standard deviation, as practiced in the art. Alternatively, "about" may mean a range of up to 5% (i.e., ±5%), for example, fluctuating within a particular numerical range given ± 2%, ± 1% or ± 0.5%. Where a particular value is given in the present disclosure or in the claims, unless otherwise stated, "about" should be considered to mean being within an acceptable error range for that particular value.

The term "identity" is also known as consistency. The percent identity between two sequences is a function of identical positions shared by the sequences (i.e., %identity = number of identical positions/total number of positions × 100), where the number of gaps that need to be introduced to produce an optimal alignment of the two sequences and the length of each gap should be taken into consideration. As shown in the following non-limiting examples, comparison of sequences and determination of percent identity between two sequences can be accomplished using a mathematical algorithm. The percent identity between two amino acid sequences can be determined using the algorithm of E. Meyers and W. Miller (Comput. Appl. Biosci., 4:11-17 (1988)), which has been incorporated into the ALIGN program (version 2.0) and uses a PAM120 residue weight table with a gap length penalty of 12 and a gap penalty of 4. In addition, the percent identity of two amino acid sequences can be determined using the algorithm of Needleman and Wunsch (J. Mol. Biol., 484-453 (1970)), which has been incorporated into the GAP program in the GCG software package (available at www.gcg.com) and uses the Blossum 62 matrix or the PAM250 matrix with a gap weight of 16, 14, 12, 10, 8, 6 or 4 and a length weight of 1, 2, 3, 4, 5 or 6.

The terms "Xn" and "Xaa" are equivalent and refer to an unspecified amino acid whose scope is specified by the subsequent definitions in the relevant description.

Unless otherwise indicated herein, the term "include" or equivalents thereof (e.g., comprise and contain) is an openended term. It means including but not limited to the stated elements, steps or components but is open to the elements, steps or components not explicitly stated.

Unless otherwise stated herein, singular terms encompass plural forms, and vice versa.

All patents, patent applications and other identified publications are expressly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art. Various aspects of the present disclosure will be described in further detail in the following sections.

*Antigen-Binding Construct*

The present disclosure provides an antigen-binding construct, which comprises a first antigen-binding fragment that is monovalent and specifically binds to ECD4 antigen of HER2 on an HER2-expressing cell, wherein the first antigen-binding fragment is an scFv; a heavy chain variable region of the first antigen-binding fragment comprises an amino acid mutation at position 30 according to the Kabat numbering system (in some examples, the amino acid at position 30 is mutated into E); or/and a light chain variable region of the first antigen-binding fragment comprises an amino acid mutation at position 53 according to the Kabat numbering system (in some examples, the amino acid at position 53 is mutated into Y).

In some embodiments, the antigen-binding construct described herein further comprises a second antigen-binding fragment that is monovalent and specifically binds to ECD2 antigen of HER2 on an HER2-expressing cell.

In some embodiments, the antigen-binding construct provided herein comprises an immunoglobulin functional domain operably linked to a first antigen-binding fragment or/and a second antigen-binding fragment, e.g., one or more of the CL region, the CH1 region, the CH2 region, and/or the CH3 region of an antibody.

In some embodiments, the antigen-binding construct provided herein comprises a constant region operably linked to a first antigen-binding fragment or/and a second antigen-binding fragment. The constant region may be a natural sequence constant region or a mutated constant region of an immunoglobulin, e.g., one or more of natural or mutated CL, CH1, CH2, and/or CH3 functional domains; in some examples, these functional domains are operably linked in a manner conventional in the art. The constant region may be derived from a constant region of a human immunoglobulin, such as from IgG1, IgG2, IgG3 or IgG4. In some examples, the constant region may have modifications so that its ability to mediate effector functions is improved.

In some embodiments, the antigen-binding construct provided herein comprises an immunoglobulin functional domain or skeleton, e.g., an Fc, operably linked to a first antigen-binding fragment or/and a second antigen-binding fragment. The term Fc includes natural sequence Fc regions and variant Fc regions. The Fc may be a human Fc; for example, it is derived from IgG1, IgG2, IgG3 or IgG4. The Fc may have modifications so that its ability to mediate effector functions is improved. For example, in some embodiments, the skeleton has modifications, such as knob into hole, H435R, Y436F and defucosylation.

The antigen-binding construct provided herein may be monovalent, bivalent or polyvalent, and may be monospecific, bispecific or multispecific. In some embodiments, the antigen-binding construct provided herein is an antibody.

It has been unexpectedly found that the introduction of mutations into the variable region of the scFV form of the first antigen-binding fragment of the antigen-binding construct can reduce the aggregation of the antigen-binding construct. These mutations may be, for example, mutation of the amino acid at position 30 (Kabat number) in the heavy chain variable region of the first antigen-binding fragment into negatively charged glutamic acid (E), or mutation of the amino acid at position 53 (Kabat number) in the light chain variable region of the first antigen-binding fragment into electrically neutral tyrosine (Y), or both the mutations are introduced. In addition, the inventors found that constructing a bispecific antigen-binding construct directly from the original unmutated (the amino acid at position 30 is not E or/and the amino acid at position 53 is not Y) scFV form of the first antigen-binding fragment will cause its undesirable aggregation property to be given to the bispecific antigen-binding construct; however, after the introduction of the mutations described herein into the scFV, the constructed bispecific antibody showed excellent resistance to aggregation. In some embodiments, the introduction of the mutations does not significantly reduce the affinity of an antigen-binding construct (e.g., a monospecific or bispecific antigen-binding construct) for the HER2 antigen. In some embodiments, the antigen-binding construct has good affinity for the HER2 antigen. In some embodiments, the bispecific antigen-binding constructs (23C2 Her2-2, etc.) show higher binding affinity than trastuzumab and pertuzumab for antigen Her2. In some embodiments, the antigen-binding construct has a KD of about 1E-8 M or less, about 1E-9 M or less, about 6.11E-10 M or less, about IE-11 M or less, or about 1E-11 M or less, for the Her2 antigen. In some embodiment, the Her2 antigen is human Her2 antigen. In some embodiments, the binding affinity KD of the antigen-binding construct provided herein is measured by Biacore.

In some embodiments, the antigen-binding construct described herein also shows excellent anti-tumor properties such as killing tumor cells or/and inhibiting tumor growth. In some embodiments, the antigen-binding constructs constructed herein have good killing effects on a variety of tumor cells, or/and a variety of cells expressing HER2 at different levels. For example, some of the antigen-binding constructs have good killing effects on BT474 (Her2+++) tumor cells; some of the antigen-binding constructs have good killing effects on NCI-N87 (Her2++) tumor cells; some of the antigen-binding constructs have good killing effects on trastuzumab-resistant tumor cells (e.g., JIMT-1); and some of the antigen-binding constructs have good killing effects on tumor cells expressing Her2 at low level (e.g., MCF-7 Her2 0/+).

In some embodiments, it is found by experiment with a mouse model that the antigen-binding construct described herein has no significant toxic effects.

*Bispecific Antigen-Binding Construct*

Provided herein is a bispecific antigen-binding construct that binds to HER2. The bispecific antigen-binding construct comprises two antigen-binding fragments, each of which binds to a specific domain or epitope of HER2. The antigen-binding construct provided herein is a bispecific antigen-binding construct, which comprises a first antigen-binding fragment that is monovalent and specifically binds to ECD4 antigen of HER2 on HER2-expressing cells, and a second antigen-binding fragment that is monovalent and specifically binds to ECD2 antigen of HER2 on HER2-expressing cells;
wherein the first antigen-binding fragment is an scFv; a heavy chain variable region of the first antigen-binding fragment comprises an amino acid mutation at position 30 according to the Kabat numbering system and the amino acid is mutated into E; or/and a light chain variable region of the first antigen-binding fragment comprises an amino acid mutation at position 53 according to the Kabat numbering system and the amino acid is mutated into Y.

In some embodiments, the antigen-binding construct provided herein is a bispecific antigen-binding construct, wherein a heavy chain variable region of the first antigen-binding fragment comprises an amino acid mutation at position 30 according to the Kabat numbering system, and the amino acid is mutated into E.

In some embodiments, the antigen-binding construct provided herein is a bispecific antigen-binding construct, wherein a light chain variable region of the first antigen-binding fragment comprises an amino acid mutation at position 53 according to the Kabat numbering system, and the amino acid is mutated into Y.

In some embodiments, the antigen-binding construct provided herein is a bispecific antigen-binding construct, wherein a heavy chain variable region of the first antigen-binding fragment comprises an amino acid mutation at position 30 according to the Kabat numbering system and the amino acid is mutated into E; and a light chain variable region of the first antigen-binding fragment comprises an amino acid mutation at position 53 according to the Kabat numbering system and the amino acid is mutated into Y.

In some embodiments, a heavy chain variable region of the first antigen-binding fragment comprises an amino acid mutation at position 30 according to the Kabat numbering system, and the amino acid before being mutated is not E; in some embodiments, the amino acid before being mutated is K, that is, in some embodiments, the heavy chain variable region of the first antigen-binding fragment comprises a K30E mutation according to the Kabat numbering system.

In some embodiments, a light chain variable region of the first antigen-binding fragment comprises an amino acid mutation at position 53 according to the Kabat numbering system, and the amino acid before being mutated is not Y; in some embodiments, the amino acid before being mutated is F, that is, in some embodiments, the light chain variable region of the first antigen-binding fragment comprises an F53Y mutation according to the Kabat numbering system.

In some embodiments, the antigen-binding construct described herein is a bispecific antigen-binding construct, wherein the first antigen-binding fragment comprises a heavy chain CDR1, a heavy chain CDR2 and a heavy chain CDR3, wherein the heavy chain CDR1, the heavy chain CDR2 and the heavy chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity or at least 99% identity to the amino acid sequences according to SEQ ID NOs: 27, 28 and 29, respectively. In some specific embodiments, the antigen-binding construct described herein is a bispecific antigen-binding construct, wherein the first antigen-binding fragment comprises a heavy chain CDR1, a heavy chain CDR2 and a heavy chain CDR3, wherein the heavy chain CDR1, the heavy chain CDR2 and the heavy chain CDR3 comprise the amino acid sequences according to SEQ ID NOs: 27, 28 and 29, respectively.

In some embodiments, the antigen-binding construct described herein is a bispecific antigen-binding construct, wherein the first antigen-binding fragment comprises a light chain CDR1, a light chain CDR2 and a light chain CDR3, wherein the light chain CDR1, the light chain CDR2 and the light chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity or at least 99% identity to the amino acid sequences according to SEQ ID NOs: 30, 34 and 32, respectively. In some specific embodiments, the antigen-binding construct described herein is a bispecific antigen-binding construct, wherein the first antigen-binding fragment comprises a light chain CDR1, a light chain CDR2 and a light chain CDR3, wherein the light chain CDR1, the light chain CDR2 and the light chain CDR3 comprise the amino acid sequences according to SEQ ID NOs: 30, 34 and 32, respectively.

In some embodiments, the antigen-binding construct described herein is a bispecific antigen-binding construct, wherein the first antigen-binding fragment comprises a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity or at least 99% identity to the amino acid sequences according to SEQ ID NOs: 27, 28 and 29, respectively; the light chain CDR1, the light chain CDR2 and the light chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity or at least 99% identity to the amino acid sequences according to SEQ ID NOs: 30, 34 and 32, respectively. In some embodiments, the antigen-binding construct described herein is a bispecific antigen-binding construct, wherein the first antigen-binding fragment comprises a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 27, 28 and 29, respectively; and the light chain CDR1, the light chain CDR2 and the light chain CDR3 comprise the amino acid sequences according to SEQ ID NOs: 30, 34 and 32, respectively. In some examples, the first antigen-binding fragment comprises the heavy chain CDR1, the heavy chain CDR2, the heavy chain CDR3, the light chain CDR1, the light chain CDR2 and the light chain CDR3 according to SEQ ID NOs: 43, 28, 29, 30, 31 and 32, respectively. In some examples, the first antigen-binding fragment comprises the heavy chain CDR1, the heavy chain CDR2, the heavy chain CDR3, the light chain CDR1, the light chain CDR2 and the light chain CDR3 according to SEQ ID NOs: 43, 28, 29, 30, 33 and 32, respectively. In some examples, the first antigen-binding fragment comprises the heavy chain CDR1, the heavy chain CDR2, the heavy chain CDR3, the light chain CDR1, the light chain CDR2 and the light chain CDR3 according to SEQ ID NOs: 44, 28, 29, 30, 31 and 32, respectively. In some embodiments, the antigen-binding construct described herein is a bispecific antigen-binding construct, wherein the first antigen-binding fragment comprises a mutant of a heavy chain variable region of trastuzumab and a light chain variable region of trastuzumab, wherein the mutant of the heavy chain variable region comprises a K30E mutation according to the Kabat numbering system.

In some embodiments, the antigen-binding construct described herein is a bispecific antigen-binding construct, wherein the first antigen-binding fragment comprises a heavy chain variable region of trastuzumab and a mutant of a light chain variable region of trastuzumab, wherein the mutant of the light chain variable region comprises an F53Y mutation according to the Kabat numbering system.

In some embodiments, the antigen-binding construct described herein is a bispecific antigen-binding construct, wherein the first antigen-binding fragment comprises a mutant of a heavy chain variable region of trastuzumab and a mutant of a light chain variable region of trastuzumab, wherein the mutant of the heavy chain variable region comprises a K30E mutation, and the mutant of the light chain variable region comprises an F53Y mutation.

The first antigen-binding fragment is an scFV comprising a heavy chain variable region and a light chain variable region. In some embodiments, the heavy chain variable region is the heavy chain variable region of trastuzumab or the mutant thereof, and the light chain variable region is the light chain variable region of trastuzumab or the mutant thereof.

In some embodiments, the antigen-binding construct described herein is a bispecific antigen-binding construct, wherein the heavy chain variable region and the light chain variable region of the first antigen-binding fragment comprise amino acid sequences having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity or at least 99% identity to the amino acid sequences according to SEQ ID NOs: 41 and 42, respectively. In some embodiments, the first antigen-binding fragment comprises any of the CDR region sequence characteristics described above. In some embodiments, the first antigen-binding fragment comprises a heavy chain variable region and a light chain variable region, wherein the heavy chain variable region and the light chain variable region comprise the amino acid sequences according to SEQ ID NOs: 41 and 42, respectively. In some embodiments, the first antigen-binding fragment comprises a heavy chain variable region and a light chain variable region, wherein the heavy chain variable region and the light chain variable region comprise the amino acid sequences according to SEQ ID NOs: 35 and 36, respectively. In some embodiments, the first antigen-binding fragment comprises a heavy chain variable region and a light chain variable region, wherein the heavy chain variable region and the light chain variable region comprise the amino acid sequences according to SEQ ID NOs: 35 and 39, respectively. In some embodiments, the first antigen-binding fragment comprises a heavy chain variable region and a light chain variable region, wherein the heavy chain variable region and the light chain variable region comprise the amino acid sequences according to SEQ ID NOs: 40 and 36, respectively.

In some embodiments, the heavy chain variable region and the light chain variable region of the first antigen-binding fragment are linked by a linker to form an scFV The linker may be any suitable, e.g. electrically charged and/or flexible, linker. In a specific embodiment, the linker consists of 1 to 50 amino acids linked by peptide bonds, wherein the amino acids may be selected from the group consisting of the 20 naturally occurring amino acids; in a more preferred embodiment, the 1 to 50 amino acids are selected from the group consisting of glycine, alanine, proline, asparagine, glutamine and lysine; even more preferably, the linker consists of mostly sterically unhindered amino acids (such as glycine and alanine). A particularly useful flexible linker is (GGGGS)n (also known as (G4S)n). In some embodiments, n is any number between 1 and 10, i.e., 1, 2, 3, 4, 5, 6, 7, 8, 9, and 10, or any range defined by any two of the foregoing numbers, e.g., 1-5, 2-5, 3-6, 2-4 and 1-4. In some specific embodiments, the linker comprises a flexible linker peptide having a sequence of GGSGGSGGSGGSGG (SEQ ID NO: 52). In some specific embodiments, the linker comprises a flexible linker peptide having a sequence of GGGGSGGGGSGGGGS (SEQ ID NO: 53). In some specific embodiments, the linker comprises a flexible linker peptide having a sequence of GGGGSGGGGSGGGGSGGGGS (SEQ ID NO: 54). In some specific embodiments, the linker comprises a flexible linker peptide having a sequence of GGGPGKR (SEQ ID NO: 55). Examples of other suitable linkers also include those disclosed in Table 4 of the Patent Application No. WO2019195535.

In some embodiments, the antigen-binding construct described herein is a bispecific antigen-binding construct, wherein a VH and a VL of the first antigen-binding fragment are arranged from N-terminus to C-terminus in the following order: VH-linker-VL. In some embodiments, the VH and the VL of the first antigen-binding fragment are arranged from N-terminus to C-terminus in the following order: VL-linker-VH.

In some embodiments, the antigen-binding construct described herein is a bispecific antigen-binding construct, wherein the second antigen-binding fragment is an Fab. The second antigen-binding fragment specifically binds to ECD2 antigen of HER2 on an HER2-expressing cell.

In some embodiments, the second antigen-binding fragment comprises a heavy chain CDR1, a heavy chain CDR2 and a heavy chain CDR3 of pertuzumab. In some embodiments, the second antigen-binding fragment comprises a light chain CDR1, a light chain CDR2 and a light chain CDR3 of pertuzumab. In some embodiments, the second antigen-binding fragment comprises a heavy chain CDR1, a heavy chain CDR2, a heavy chain CDR3, a light chain CDR1, a light chain CDR2 and a light chain CDR3 of pertuzumab. The amino acid sequences of the heavy chain CDR1, the heavy chain CDR2, the heavy chain CDR3, the light chain CDR1, the light chain CDR2 and the light chain CDR3 of pertuzutnab are: GFTFTDYTMD (SEQ ID NO: 45), DVNPNSGGSIYNQRFKG (SEQ ID NO: 46), NLGPSFYFDY (SEQ ID NO: 47), KASQDVSIGVA (SEQ ID NO: 48), SASYRYT (SEQ ID NO: 49), and QQYYIYPYT (SEQ ID NO: 50), respectively.

In some embodiments, the second antigen-binding fragment comprises a heavy chain variable region and a light chain variable region of pertuzutnab. In some embodiments, the second antigen-binding fragment is an Fab fragment of pertuzumab. The amino acid sequence of the heavy chain variable region of pertuzumab is set forth in SEQ ID NO: 37, and the amino acid sequence of the light chain variable region of pertuzumab is set forth in SEQ ID NO: 38.

In some embodiments, the antigen-binding construct described herein is a bispecific antigen-binding construct comprising a constant region. In some embodiments, the constant region is linked to the first antigen-binding fragment and the second antigen-binding fragment by a linker. In some embodiments, the constant region is directly linked to the first antigen-binding fragment and the second antigen-binding fragment.

In some embodiments, the antigen-binding construct described herein is a bispecific antigen-binding construct comprising a skeleton. In some embodiments, the skeleton is linked to the first antigen-binding fragment and the second antigen-binding fragment by a linker polypeptide (e.g., an immunoglobulin hinge region polypeptide selected from the group consisting of IgG1, IgG2 and IgG4 hinge regions). In some embodiments, the skeleton is directly linked to the first antigen-binding fragment and the second antigen-binding fragment.

In some embodiments, the skeleton is a dimeric Fc comprising a first Fc polypeptide and a second Fc polypeptide. The Fc described herein is used to define the C-terminal region of an immunoglobulin heavy chain comprising at least one portion of a constant region. An Fc polypeptide of a dimeric Fc refers to one of two polypeptides from which a dimeric Fc domain is formed, i.e. a polypeptide comprising the C-terminal constant region of an immunoglobulin heavy chain and capable of stable self-association. Unless otherwise stated herein, the amino acid residues in the Fc region or constant region are numbered according to the EU numbering system. For example, the Fc polypeptide of dimeric IgG Fc comprises IgG CH2 and IgG CH3 constant domain sequences. The CH3 domain comprises two CH3 sequences, which are from a first Fc polypeptide and a second Fc polypeptide, respectively, and the CH2 domain comprises two CH2 sequences, which are from a first Fc polypeptide and a second Fc polypeptide, respectively. In some embodiments, the first antigen-binding fragment is operably linked to the first Fc polypeptide, and the second antigen-binding fragment is operably linked to the second Fc polypeptide. In some embodiments, the first antigen-binding fragment is operably linked to the first Fc polypeptide by a first polypeptide linker, and the second antigen-binding fragment is operably linked to the second Fc polypeptide by a second polypeptide linker. The first linker polypeptide and the second linker polypeptide can form a covalent bond, such as a disulfide bond. In some embodiments, the first linker polypeptide or/and the second linker polypeptide comprise the hinge region of IgG1; in some embodiments, the first linker polypeptide or/and the second linker polypeptide comprises the hinge region of IgG2; in some embodiments, the first linker polypeptide or/and the second linker polypeptide comprises the hinge region of IgG3; in some embodiments, the first linker polypeptide or/and the second linker polypeptide comprises the hinge region of IgG4.

In some embodiments, the dimeric Fc is an Fc of human IgG1. In some embodiments, the dimeric Fc is an Fc of human IgG4.

In some embodiments, the dimeric Fc has a modification. In some embodiments, the dimeric Fc has an H435R modification or/and a Y436F modification, which may occur in either polypeptide chain of the first Fc polypeptide and the second Fc polypeptide. In some embodiments, the dimeric Fc has an H435R modification or/and a Y436F modification, which occur in only one Fc polypeptide rather than in the other Fc polypeptide. In some embodiments, the dimeric Fc has mutation sites of knob-into-hole, such as Y349C, T366S, L368A, Y407V, S354C and T366W. In some embodiments, one chain of the dimeric Fc has T366Y/W or/and S354C, and the other chain has Y407T/V, Y349C, T366S or/and L368A.

In some embodiments, the dimeric Fc does not contain fucose.

In some embodiments, the antigen-binding construct described herein is glycosylated.

In some embodiments, the antigen-binding construct described herein is afucosylated. The interaction of IgG1 with FcgRIIIa can be improved by defucosylation, and thereby the ADCC of the antibody is enhanced. Methods of producing antigen-binding constructs with little or no fucose at the Fc glycosylation site without altering the amino acid sequence are known in the art, for example, adjusting the composition of the medium the expressing cells are in, or knocking out fucose expression-related genes such as FUT8 in the expressing cells.

In some embodiments, the bispecific antigen-binding construct provided herein is bivalent.

Bispecific antigen-binding constructs are illustratively provided in Table S1. In some embodiments, the bispecific antigen-binding construct described herein is a bispecific antibody. Bispecific antibodies, such as Expi Her2-2, Expi Her2-3, Expi Her2-4, 23C-HER2-2, 23C-HER2-3 and 23C-HER2-4, are illustratively provided in Table S2, and the sequences of CDRs and variable regions are provided in Table S2. Exemplary bispecific antibodies comprise two heavy chains and one light chain. The amino acid sequences of the heavy chains of Expi Her2-2 and 23C-HER2-2 are set forth in SEQ ID NO: 11 and SEQ ID NO: 13, and the amino acid sequences of the light chains of Expi Her2-2 are set forth in SEQ ID NO: 15; the amino acid sequences of the heavy chains of Expi Her2-3 and 23C-HER2-3 are set forth in SEQ ID NO: 21 and SEQ ID NO: 13, and the amino acid sequences of the light chains of Expi Her2-3 and 23C-HER2-3 are set forth in SEQ ID NO: 15; the amino acid sequences of the heavy chains of Expi Her2-4 and 23C-HER2-4 are set forth in SEQ ID NO: 23 and SEQ ID NO: 13, and the amino acid sequences of the light chains of Expi Her2-4 and 23C-HER2-4 are set forth in SEQ ID NO: 15.

Further provided herein are some ADCC-enhanced bispecific antigen-binding constructs. Provided herein are exemplary ADCC-enhanced bispecific antibodies such as 23C-HER2-2, 23C-HER2-3 and 23C-HER2-4.

Table S1: Exemplary bispecific antigen-binding constructs

| Name | Description | First antigen-binding fragment | Second antigen-binding fragment |
|---|---|---|---|
| / | Epitope-containing domain | ECD4 | ECD2 |
| | Form | scFV | Fab |
| | Original sequence | Trastuzumab | Pertuzumab |
| 1 | Sequence substitution | VL: F53Y | / |
| 2 | Sequence substitution | VH: K30E | / |
| 3 | Sequence substitution | VL: F53Y<br>VH: K30E | / |

Table S2: CDR and variable region sequences of exemplary bispecific antibodies

| Name | | Description | Amino acid sequence |
|---|---|---|---|
| Expi Her2-2/ 23C-HER2-2 | First antigen-binding fragment (scFV) | Heavy chain CDR1 | GFNIEDTYIH (SEQ ID NO: 43) |
| | | Heavy chain CDR2 | RIYPTNGYTRYADSVKG (SEQ ID NO: 28) |
| | | Heavy chain CDR3 | WGGDGFYAMDYW (SEQ ID NO: 29) |
| | | Heavy chain variable region | EVQLVESGGGLVQPGGSLRLSCAASGFNIEDTYIHWVRQAPGKGLE WVARIYPTNGYTRYADSVKGRFTISADTSKNTAYLQMNSLRAEDTA VYYCSRWGGDGFYAMDYWGQGTLVTVSS (SEQ ID NO: 35) |
| | | Light chain CDR1 | CRASQDVNTAVAW (SEQ ID NO: 30) |
| | | Light chain CDR2 | SASYLYS (SEQ ID NO: 31) |
| | | Light chain CDR3 | QQHYTTPPT (SEQ ID NO: 32) |
| | | Light chain variable region | DIQMTQSPSSLSASVGDRVTITCRASQDVNTAVAWYQQKPGKAPKL LIYSASYLYSGVPSRFSGSRSGTDFTLTISSLQPEDFATYYCQQHYTTP PTFGQGTKVEIK (SEQ ID NO: 36) |
| | Second antigen-binding fragment (Fab) | Heavy chain variable region | EVQLVESGGGLVQPGGSLRLSCAASGFTFTDYTMDWVRQAPGKGL EWVADVNPNSGGSIYNQRFKGRFTLSVDRSKNTLYLQMNSLRAEDT AVYYCARNLGPSFYFDYWGQGTLVTVSS (SEQ ID NO: 37) |
| | | Light chain variable region | DIQMTQSPSSLSASVGDRVTITCKASQDVSIGVAWYQQKPGKAPKLL IYSASYRYTGVPSRFSGSGSGTDFTLTISSLQPEDFATYYCQQYYIYPY TFGQGTKVEIK (SEQ ID NO: 38) |

| Name | | Description | Amino acid sequence |
|---|---|---|---|
| Expi Her2-3/ 23C-HER2-3 | First antigen-binding fragment (scFV) | Heavy chain CDR1 | GFNIEDTYIH (SEQ ID NO: 43) |
| | | Heavy chain CDR2 | RIYPTNGYTRYADSVKG (SEQ ID NO: 28) |
| | | Heavy chain CDR3 | WGGDGFYAMDYW 29) (SEQ ID NO: |
| | | Heavy chain variable region | EVQLVESGGGLVQPGGSLRLSCAASGFNIEDTYIHWVRQAPGKGLE WVARIYPTNGYTRYADSVKGRFTISADTSKNTAYLQMNSLRAEDTA VYYCSRWGGDGFYAMDYWGQGTLVTVSS (SEQ ID NO: 35) |
| | | Light chain CDR1 | CRASQDVNTAVAW (SEQ ID NO: 30) |
| | | Light chain CDR2 | SASFLYS (SEQ ID NO: 33) |
| | | Light chain CDR3 | QQHYTTPPT (SEQ ID NO: 32) |
| | | Light chain | DIQMTQSPSSLSASVGDRVTITCRASQDVNTAVAWYQQKPGKAPKL |
| | Second antigen-binding fragment (Fab) | variable region | LIYSASFLYSGVPSRFSGSRSGTDFTLTISSLQPEDFATYYCQQHYTTP PTFGQGTKVEIK (SEQ ID NO: 39) |
| | | Heavy chain variable region | EVQLVESGGGLVQPGGSLRLSCAASGFTFTDYTMDWVRQAPGKGL EWVADVNPNSGGSIYNQRFKGRFTLSVDRSKNTLYLQMNSLRAEDT AVYYCARNLGPSFYFDYWGQGTLVTVSS (SEQ ID NO: 37) |
| | | Light chain variable region | DIQMTQSPSSLSASVGDRVTITCKASQDVSIGVAWYQQKPGKAPKLL IYSASYRYTGVPSRFSGSGSGTDFTLTISSLQPEDFATYYCQQYYIYPY TFGQGTKVEIK (SEQ ID NO: 38) |

EP 4 144 760 A1

| Name | | Description | Amino acid sequence |
|---|---|---|---|
| Expi Her2-4/ 23C-HER2-4 | First antigen-binding fragment (scFV) | Heavy chain CDR1 | GFNIKDTYIH　(SEQ ID NO: 44) |
| | | Heavy chain CDR2 | RIYPTNGYTRYADSVKG　(SEQ ID NO: 28) |
| | | Heavy chain CDR3 | WGGDGFYAMDYW 29)　(SEQ ID NO: |
| | | Heavy chain variable region | EVQLVESGGGLVQPGGSLRLSCAASGFNIKDTYIHWVRQAPGKGLE WVARIYPTNGYTRYADSVKGRFTISADTSKNTAYLQMNSLRAEDTA VYYCSRWGGDGFYAMDYWGQGTLVTVSS　(SEQ ID NO: 40) |
| | | Light chain CDR1 | CRASQDVNTAVAW 30)　(SEQ ID NO: |
| | | Light chain CDR2 | SASYLYS　(SEQ ID NO: 31) |
| | | Light chain CDR3 | QQHYTTPPT　(SEQ ID NO: 32) |
| | | Light chain variable region | DIQMTQSPSSLSASVGDRVTITCRASQDVNTAVAWYQQKPGKAPKL LIYSASYLYSGVPSRFSGSRSGTDFTLTISSLQPEDFATYYCQQHYTTP PTFGQGTKVEIK　(SEQ ID NO: 36) |
| | Second antigen-binding fragment (Fab) | Heavy chain variable region | EVQLVESGGGLVQPGGSLRLSCAASGFTFTDYTMDWVRQAPGKGL EWVADVNPNSGGSIYNQRFKGRFTLSVDRSKNTLYLQMNSLRAEDT AVYYCARNLGPSFYFDYWGQGTLVTVSS　(SEQ ID NO: 37) |
| | | Light chain variable region | DIQMTQSPSSLSASVGDRVTITCKASQDVSIGVAWYQQKPGKAPKLL IYSASYRYTGVPSRFSGSGSGTDFTLTISSLQPEDFATYYCQQYYIYPY TFGQGTKVEIK　(SEQ ID NO: 38) |
| Monospecific Antigen-Binding Construct | | | |

A monospecific antigen-binding construct refers to an antigen-binding construct that has one type of binding specificity.

In some embodiments, the antigen-binding construct provided herein is a monospecific antigen-binding construct, which is a first antigen-binding fragment that is monovalent and specifically binds to ECD4 antigen of HER2 on an HER2-expressing cell, wherein the first antigen-binding fragment is an scFv; a heavy chain variable region of the first antigen-binding fragment comprises an amino acid mutation at position 30 according to the Kabat numbering system and the amino acid is mutated into E; or/and a light chain variable region of the first antigen-binding fragment comprises an amino acid mutation at position 53 according to the Kabat numbering system and the amino acid is mutated into Y.

In some embodiments, the antigen-binding construct provided herein is a monospecific antigen-binding construct, which is bivalent and comprises two of the first antigen-binding fragments that are monovalent and specifically bind to ECD4 antigen of HER2 on an HER2-expressing cell.

In some embodiments, the antigen-binding construct provided herein is a monospecific antigen-binding construct, the first antigen-binding fragment of which may has the sequence characteristics of the first antigen-binding fragment of the bispecific antigen-binding construct described above.

In some embodiments, the antigen-binding construct provided herein is a monospecific antigen-binding construct, which is bivalent and comprises an Fc operably linked to the first antigen-binding fragment. In some embodiments, the Fc is an Fc of human IgG1.

In some embodiments, the antigen-binding construct is a bivalent monospecific antibody, which comprises the amino acid sequence of SEQ ID NO: 3.

In some embodiments, the antigen-binding construct is a bivalent monospecific antibody, which comprises the amino acid sequence of SEQ ID NO: 9.

In some embodiments, the antigen-binding construct is a bivalent monospecific antibody, which comprises the amino acid sequence of SEQ ID NO: 51.

*Composition*

Provided herein is a pharmaceutical composition, which comprises an antigen-binding construct, or a nucleic acid encoding the antigen-binding construct, and further comprises one or more pharmaceutically acceptable carriers. In some embodiments, the composition comprises one or more of bispecific antibodies such as Expi Her2-2, Expi Her2-3, Expi Her2-4, 23C-HER2-2, 23C-HER2-3 and 23C-HER2-4, and a pharmaceutically acceptable carrier. The pharmaceutically acceptable carriers include, for example, excipients, diluents, encapsulating materials, fillers, buffering agents or other agents.

In the composition, the antigen-binding construct may be fully afucosylated (meaning that it contains no detectable fucose) or may be partially afucosylated. In some embodiments, about 20% or less, about 18% or less, about 16% or less, about 14% or less, about 12% or less, about 10% or less, about 8% or less, about 6% or less, about 5% or less, about 3% or less, about 2% or less, about 1.5% or less, about 1% or less, about 0.9% or less, about 0.8% or less, about 0.7% or less, about 0.6% or less, about 0.5% or less, about 0.4% or less, about 0.3% or less, about 0.2% or less, about 0.1% or less or about 0% of the antigen-binding construct contains no fucose. In some embodiments, about 0 to about 20%, about 0 to about 18%, about 0 to about 16%, about 0 to about 14%, about 0 to about 12%, about 0 to about 10%, about 0 to about 8%, about 0 to about 6%, about 0 to about 5%, about 0 to about 3%, about 0 to about 2%, about 0 to about 1.5%, about 0 to about 1%, about 0 to about 0.9%, about 0 to about 0.8%, about 0 to about 0.7%, about 0 to about 0.6%, about 0 to about 0.5%, about 0 to about 0.4%, about 0 to about 0.3%, about 0 to about 0.2%, about 0 to about 0.1%, or about 0.5% to about 0.6% of the antigen-binding construct contains no fucose.

*Isolated Nucleic Acid*

Provided herein is an isolated nucleic acid or a collection of isolated nucleic acids, which comprises at least one nucleic acid sequence encoding at least one polypeptide of the antigen-binding construct described herein. In some embodiments, the nucleic acid encodes the sequence of the antigen-binding construct described herein. In some embodiments, the nucleic acid may encode an amino acid sequence comprising the first antigen-binding fragment or/and the second antigen-binding fragment of the antigen-binding construct described herein. In some other embodiments, the nucleic acid may encode an amino acid sequence comprising the antigen-binding construct heavy chain or/and the antigen-binding construct light chain described herein. The nucleic acid sequences of some antigen-binding constructs, such as antigen-binding fragments of bispecific antibodies, and heavy and light chains of antigen-binding constructs are illustratively listed in the sequence listing.

*Vector*

The present disclosure provides a vector or a collection of vectors comprising one or more of the isolated nucleic acid and the collection of nucleic acids. In some embodiments, the vector is a cloning vector; in some other embodiments, the vector is an expression vector.

The expression vector is optionally any expression vector capable of expressing the antigen-binding construct described herein.

*Host cell*

In some embodiments, the present disclosure provides a host cell comprising the vector, the host cell being a suitable host cell for cloning or encoding an antigen-binding construct. In some embodiments, the host cell is a prokaryotic cell. In other embodiments, the host cell is a eukaryotic cell. In some embodiments, the host cell is selected from the group consisting of yeast cells, mammalian cells or other cells suitable for preparing an antigen-binding construct. Mammalian cells are, for example, Chinese hamster ovary (CHO) cells or CHO-S cells.

*Method for Preparing Antigen-Binding Constructs*

In some embodiments, provided herein is a method for preparing an antigen-binding construct, which comprises: culturing a host cell comprising a nucleic acid encoding the antigen-binding construct under conditions suitable for expression of the antigen-binding construct, and recovering the antigen-binding construct from the host cell or host cell culture medium. To produce the antigen-binding construct, a nucleic acid encoding the antigen-binding construct is isolated and inserted into one or more vectors for further cloning and/or expression in a host cell. The nucleic acid can be obtained using various methods known in the art, such as gene splicing, chemical synthesis and cloning.

*Use*

Provided herein is use of the antigen-binding construct. In a specific embodiment, the antigen-binding construct used may be Expi Her2-2, Expi Her2-3, Expi Her2-4, 23C-HER2-2, 23C-HER2-3 and 23C-HER2-4.

In some embodiments, administering to a subject a therapeutically effective amount of the antigen-binding construct described herein can kill HER2-expressing tumor cells or inhibit the growth of HER2-expressing tumor cells. In some embodiments, provided herein is use of the antigen-binding construct for preparing a medicament for killing HER2-expressing tumor cells or inhibiting the growth of HER2-expressing tumor cells. In some embodiments, provided herein is an antigen-binding construct for use in killing HER2-expressing tumor cells or inhibiting the growth of HER2-expressing tumor cells. In some embodiments, the present disclosure provides a therapeutic agent for use in killing HER2-expressing tumor cells or inhibiting the growth of HER2-expressing tumor cells, which comprises the antigen-binding construct described herein as an active ingredient.

In some embodiments, an HER2-expressing tumor can be treated by administering to a subject a therapeutically effective amount of the antigen-binding construct described herein. Subjects in need of treatment include those with a disease or condition, those at risk of developing the disease or condition, and those who may have the disease or condition and whose purpose is to prevent, delay or alleviate the disease or condition. In some embodiments, provided herein is use of said antigen-binding construct for preparing a medicament for treating a subject having an HER2-expressing tumor. In some embodiments, provided herein is an antigen-binding construct for use in treating an HER2-expressing tumor. In some embodiments, provided herein is a therapeutic agent for use in treating an HER2-expressing tumor, which comprises the antigen-binding construct described herein as an active ingredient. Examples of the tumor include, but are not limited to: biliary tract cancer, carcinosarcoma, esophageal cancer, gastroesophageal junction cancer, breast cancer, gastric cancer, pancreatic cancer, head and neck cancer, colorectal cancer, renal cancer, cervical cancer, ovarian cancer, endometrial cancer, uterine cancer, malignant melanoma, pharyngeal cancer, oral cancer, and skin cancer.

In certain embodiments, the tumor is HER2 0-1+, 1+, HER2 2+ or HER2 3+, as determined by IHC. In some embodiments, the tumor is HER2 2+ or lower, or HER2 1+ or lower.

In some embodiments, administering to a cell an effective amount of an antigen-binding construct can inhibit, reduce or block HER2 signaling in the cell. In some embodiments, provided herein is use of the antigen-binding construct for preparing a medicament for inhibiting, reducing or blocking HER2 signaling in a cell. In some embodiments, provided herein is an antigen-binding construct for use in inhibiting, reducing or blocking HER2 signaling in a cell. In some embodiments, provided herein is a therapeutic agent for use in inhibiting, reducing or blocking HER2 signaling in a cell, which comprises the antigen-binding construct described herein as an active ingredient. In some embodiments, the cell is a tumor cell.

In some embodiments, provided is a method for detecting or measuring HER2 in a sample, which comprises contacting the sample with the antigen-binding construct described herein and detecting or measuring a binding complex.

*Method for Enhancing Resistance of Antigen-Binding Constructs to Aggregation*

Provided herein is a method for enhancing resistance of an antigen-binding construct to aggregation, wherein the antigen-binding construct comprises a first antigen-binding fragment that is monovalent and specifically binds to ECD4 antigen of HER2 on an HER2-expressing cell, the first antigen-binding fragment being an scFv; the method comprises modifying the antigen-binding construct so that a heavy chain variable region of the first antigen-binding fragment comprises an amino acid mutation at position 30 according to the Kabat numbering system and the amino acid is mutated into E, or/and that a light chain variable region of the first antigen-binding fragment comprises an amino acid mutation at position 53 according to the Kabat numbering system and the amino acid is mutated into Y. In some embodiments, the antigen-binding construct described herein further comprises a second antigen-binding fragment that is monovalent and specifically binds to ECD2 antigen of HER2 on an HER2-expressing cell, wherein the second antigen-binding fragment is an Fab. In some embodiments, the first antigen-binding fragment comprises a group of CDRs selected from:

> i. a group comprising a heavy chain CDR1, a heavy chain CDR2 and a heavy chain CDR3, wherein the heavy chain CDR1, the heavy chain CDR2 and the heavy chain CDR3 comprise the amino acid sequences according to SEQ ID NOs: 27, 28 and 29, respectively;
> ii. a group comprising a heavy chain CDR1, a heavy chain CDR2 and a heavy chain CDR3, wherein the heavy chain CDR1, the heavy chain CDR2 and the heavy chain CDR3 comprise amino acid sequences having at least 80% identity to the amino acid sequences according to SEQ ID NOs: 27, 28 and 29, respectively;
> iii. a group comprising a light chain CDR1, a light chain CDR2 and a light chain CDR3, wherein the light chain CDR1, the light chain CDR2 and the light chain CDR3 comprise the amino acid sequences according to SEQ ID NOs: 30, 34 and 32, respectively;
> iv. a group comprising a light chain CDR1, a light chain CDR2 and a light chain CDR3, wherein the light chain CDR1, the light chain CDR2 and the light chain CDR3 comprise amino acid sequences having at least 80% identity to the amino acid sequences according to SEQ ID NOs: 30, 34 and 32, respectively;
> v. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 27, 28 and 29, respectively; and the light chain CDR1, the light chain CDR2 and the light chain CDR3 comprise the amino acid sequences according to SEQ ID NOs: 30, 34 and 32, respectively;
> vi. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise amino acid sequences having at least 80% identity to the amino acid sequences according to SEQ ID NOs: 27, 28 and 29, respectively; and the light chain CDR1, the light chain CDR2 and the light chain CDR3 comprise amino acid sequences having at least 80% identity to the amino acid sequences according to SEQ ID NOs: 30, 34 and 32, respectively;
> vii. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 43, 28 and 29, respectively; and the light chain CDR1, the light chain CDR2 and the light chain CDR3 comprise the amino acid sequences according to SEQ ID NOs: 30, 31 and 32, respectively;
> viii. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 43, 28 and 29, respectively; and the light chain CDR1, the light chain CDR2 and the light chain CDR3 comprise the amino acid sequences according to SEQ ID NOs: 30, 33 and 32, respectively; and ix. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 44, 28 and 29, respectively; and the light chain CDR1, the light chain CDR2 and the light chain CDR3 comprise the amino acid sequences according to SEQ ID NOs: 30, 31 and 32, respectively.

In some embodiments, a heavy chain variable region of the first antigen-binding fragment comprises an amino acid mutation at position 30 according to the Kabat numbering system, and the amino acid before being mutated is not E; in some embodiments, the amino acid before being mutated is K, that is, in some embodiments, the heavy chain variable region of the first antigen-binding fragment is made to comprise a K30E mutation according to the Kabat numbering system.

In some embodiments, a light chain variable region of the first antigen-binding fragment comprises an amino acid mutation at position 53 according to the Kabat numbering system, and the amino acid before being mutated is not Y; in some embodiments, the amino acid before being mutated is F, that is, in some embodiments, the light chain variable region of the first antigen-binding fragment is made to comprise an F53Y mutation according to the Kabat numbering system.

In some embodiments, the antigen-binding construct is modified, so that the heavy chain variable region of the first antigen-binding fragment comprises a K30E mutation according to the Kabat numbering system, and the light chain variable region of the first antigen-binding fragment comprises an F53Y mutation according to the Kabat numbering system. In some embodiments, the first antigen-binding fragment is an scFv and comprises a heavy chain variable region of trastuzumab or a mutant thereof and a light chain variable region of trastuzumab or a mutant thereof; the mutant of the heavy chain variable region is made to comprise a K30E mutation, and the mutant of the light chain variable region is made to comprise an F53Y mutation.

In some embodiments, the method further comprises confirming the ability of the modified antigen-binding construct to bind to an her2 antigen. In some embodiments, the method further comprises selecting a modified antigen-binding construct that binds to an her2 antigen. In some embodiments, the affinity (e.g., KD) of the antigen-binding construct is higher than or comparable to that of an unmodified antigen-binding construct.

In some embodiments, the method further comprises selecting an antigen-binding construct with a reduced tendency to aggregate compared to an unmodified antigen-binding construct.

Although the foregoing disclosure has been described in some detail by providing illustrations and examples for the purpose of clear understanding, it will be apparent to those of ordinary skill in the art in light of the teachings of the present disclosure that certain changes and modifications can be made to the present disclosure without departing from the spirit or scope of the appended claims. The following examples are provided for illustrative purposes only and are not intended to be limiting. Those skilled in the art will readily identify a variety of noncritical parameters that may be changed or modified to produce substantially similar results.

Unless otherwise indicated, the practice of the present disclosure will use conventional methods in protein chemistry, biochemistry, the recombinant DNA technique, and pharmacology within the art. Such techniques are explained fully in the literature. See, e.g., T. E. Creighton, Proteins: Structures and Molecular Properties (W. H. Freeman and company, 1993); A. L. Lehninger, Biochemistry (Worth Publishers, Inc., currently added); Sambrook et al., Molecular Cloning: A Laboratory Manual (1989, 2nd Edition); Methods In Enzymology (Ed. S. Colowick and N. Kaplan, Academic Press, Inc.); Remington's PharmaceuticalSciences, 18th Edition (Easton, Pennsylvania: Mack Publishing Company, 1990); and Carey and Sundberg Advanced Organic Chemistry, 3rd Edition (Plenum Press) Volumes A and B (1992).

The present disclosure further provides the following specific embodiments, but the scope of the present disclosure is not limited thereto:

Embodiment 1. An antigen-binding construct, comprising a first antigen-binding fragment that is monovalent and specifically binds to ECD4 antigen of HER2 on an HER2-expressing cell, wherein the first antigen-binding fragment is an scFv; a heavy chain variable region of the first antigen-binding fragment comprises an amino acid mutation at position 30 according to the Kabat numbering system, and preferably, the amino acid is mutated into E; or/and a light chain variable region of the first antigen-binding fragment comprises an amino acid mutation at position 53 according to the Kabat numbering system, and preferably, the amino acid is mutated into Y

Embodiment 2. The antigen-binding construct according to Embodiment 1, wherein the heavy chain variable region of the first antigen-binding fragment comprises a K30E mutation according to the Kabat numbering system, or/and the light chain variable region of the first antigen-binding fragment comprises an F53Y mutation according to the Kabat numbering system.

Embodiment 3. The antigen-binding construct according to Embodiment 1 or 2, wherein the antigen-binding construct is an antibody.

Embodiment 4. The antigen-binding construct according to any one of Embodiments 1-3, wherein the first antigen-binding fragment comprises a group of CDRs selected from:

i. a group comprising a heavy chain CDR1, a heavy chain CDR2 and a heavy chain CDR3, wherein the heavy chain CDR1, the heavy chain CDR2 and the heavy chain CDR3 comprise the amino acid sequences according to SEQ ID NOs: 27, 28 and 29, respectively;
ii. a group comprising a heavy chain CDR1, a heavy chain CDR2 and a heavy chain CDR3, wherein the heavy chain CDR1, the heavy chain CDR2 and the heavy chain CDR3 comprise amino acid sequences having at least 80% identity to the amino acid sequences according to SEQ ID NOs: 27, 28 and 29, respectively;

iii. a group comprising a light chain CDR1, a light chain CDR2 and a light chain CDR3, wherein the light chain CDR1, the light chain CDR2 and the light chain CDR3 comprise the amino acid sequences according to SEQ ID NOs: 30, 34 and 32, respectively;

iv. a group comprising a light chain CDR1, a light chain CDR2 and a light chain CDR3, wherein the light chain CDR1, the light chain CDR2 and the light chain CDR3 comprise amino acid sequences having at least 80% identity to the amino acid sequences according to SEQ ID NOs: 30, 34 and 32, respectively;

v. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 27, 28 and 29, respectively; and the light chain CDR1, the light chain CDR2 and the light chain CDR3 comprise the amino acid sequences according to SEQ ID NOs: 30, 34 and 32, respectively;

vi. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise amino acid sequences having at least 80% identity to the amino acid sequences according to SEQ ID NOs: 27, 28 and 29, respectively; and the light chain CDR1, the light chain CDR2 and the light chain CDR3 comprise amino acid sequences having at least 80% identity to the amino acid sequences according to SEQ ID NOs: 30, 34 and 32, respectively;

vii. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 43, 28 and 29, respectively; and the light chain CDR1, the light chain CDR2 and the light chain CDR3 comprise the amino acid sequences according to SEQ ID NOs: 30, 31 and 32, respectively;

viii. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 43, 28 and 29, respectively; and the light chain CDR1, the light chain CDR2 and the light chain CDR3 comprise the amino acid sequences according to SEQ ID NOs: 30, 33 and 32, respectively; and

ix. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 44, 28 and 29, respectively; and the light chain CDR1, the light chain CDR2 and the light chain CDR3 comprise the amino acid sequences according to SEQ ID NOs: 30, 31 and 32, respectively.

Embodiment 5. The antigen-binding construct according to any one of Embodiments 1-4, wherein the first antigen-binding fragment comprises a heavy chain variable region of trastuzumab or a mutant thereof, and a light chain variable region of trastuzumab or a mutant thereof; the mutant of the heavy chain variable region comprises a K30E mutation according to the Kabat numbering system, and the mutant of the light chain variable region comprises an F53Y mutation according to the Kabat numbering system.

Embodiment 6. The antigen-binding construct according to any one of Embodiments 4-5, wherein the first antigen-binding fragment is selected from:

i. the first antigen-binding fragment comprising a heavy chain variable region and a light chain variable region, which comprise the amino acid sequences according to SEQ ID NOs: 41 and 42, respectively;

ii. the first antigen-binding fragment comprising a heavy chain variable region and a light chain variable region, which comprise amino acid sequences having at least 80% identity to the amino acid sequences according to SEQ ID NOs: 41 and 42, respectively;

iii. the first antigen-binding fragment comprising a heavy chain variable region and a light chain variable region, which comprise the amino acid sequences according to SEQ ID NOs: 35 and 36, respectively;

iv. the first antigen-binding fragment comprising a heavy chain variable region and a light chain variable region, which comprise the amino acid sequences according to SEQ ID NOs: 35 and 39, respectively; and

v. the first antigen-binding fragment comprising a heavy chain variable region and a light chain variable region, which comprise the amino acid sequences according to SEQ ID NOs: 40 and 36, respectively.

Embodiment 7. The antigen-binding construct according to any one of Embodiments 1-6, wherein a VH and a VL of the first antigen-binding fragment are arranged from N-terminus to C-terminus in the following order: VH-linker-VL.

Embodiment 8. The antigen-binding construct according to any one of Embodiments 1-7, wherein the antigen-binding construct further comprises a second antigen-binding fragment that is monovalent and specifically binds to ECD2 antigen of HER2 on an HER2-expressing cell.

Embodiment 9. The antigen-binding construct according to Embodiment 8, wherein the second antigen-binding fragment is an Fab.

Embodiment 10. The antigen-binding construct according to Embodiment 8 or 9, wherein the second antigen-binding fragment comprises a heavy chain CDR1, a heavy chain CDR2, a heavy chain CDR3, a light chain CDR1, a light chain CDR2 and a light chain CDR3 of pertuzumab.

Embodiment 11. The antigen-binding construct according to Embodiment 8 or 9, wherein the second antigen-binding fragment comprises a heavy chain variable region of pertuzumab and a light chain variable region of pertuzumab.

Embodiment 12. The antigen-binding construct according to any one of Embodiments 8-11, wherein the antigen-binding construct comprises an immunoglobulin functional domain operably linked to the first antigen-binding fragment or/and the second antigen-binding fragment, the immunoglobulin functional domain comprising: i. one or more of a CL, a CH1, a CH2 and a CH3; or ii. an Fc.

Embodiment 13. The antigen-binding construct according to Embodiment 12, wherein the CL, CH1, CH2, CH3 and Fc are derived from CL, CH1, CH2, CH3 and Fc of human IgG, respectively.

Embodiment 14. The antigen-binding construct according to Embodiment 12, wherein the CL, CH1, CH2, CH3 or Fc has a modification or does not have a modification; preferably, the modification the CH3 or Fc has is, e.g., an amino acid substitution at position 435 or/and position 436 according to the Kabat numbering system.

Embodiment 15. The antigen-binding construct according to Embodiment 12, wherein the Fc is a dimeric Fc comprising a first Fc polypeptide and a second Fc polypeptide; the first antigen-binding fragment is operably linked to the first Fc polypeptide, and the second antigen-binding fragment is operably linked to the second Fc polypeptide.

Embodiment 16. The antigen-binding construct according to any one of Embodiments 1-15, wherein the antigen-binding construct is a bispecific antibody or a multispecific antibody.

Embodiment 17. The antigen-binding construct according to any one of Embodiments 1-15, wherein the antigen-binding construct is bivalent or polyvalent.

Embodiment 18. The antigen-binding construct according to any one of Embodiments 1-7, wherein the antigen-binding construct is a monospecific antibody; wherein the antigen-binding construct is monovalent, bivalent or polyvalent.

Embodiment 19. The antigen-binding construct according to Embodiment 1, wherein the antigen-binding construct is selected from the following group:

    i. the antigen-binding construct is a bivalent bispecific antibody, comprising: a heavy chain comprising SEQ ID NO: 11, a heavy chain comprising SEQ ID NO: 13, and a light chain comprising SEQ ID NO: 15;
    ii. the antigen-binding construct is a bivalent bispecific antibody, comprising: a heavy chain comprising SEQ ID NO: 21, a heavy chain comprising SEQ ID NO: 13, and a light chain comprising SEQ ID NO: 15;
    iii. the antigen-binding construct is a bivalent bispecific antibody, comprising: a heavy chain comprising SEQ ID NO: 23, a heavy chain comprising SEQ ID NO: 13, and a light chain comprising SEQ ID NO: 15;
    iv. the antigen-binding construct is a bivalent monospecific antibody, comprising the amino acid sequence of SEQ ID NO: 3;
    v. the antigen-binding construct is a bivalent monospecific antibody, comprising the amino acid sequence of SEQ ID NO: 9; and
    vi. the antigen-binding construct is a bivalent monospecific antibody, comprising the amino acid sequence of SEQ ID NO: 51.

Embodiment 20. The antigen-binding construct according to any one of Embodiments 1-19, wherein the antigen-binding construct has reduced aggregation compared to antigen-binding constructs in which the amino acid at position 30 is not E or/and the amino acid at position 53 is not Y; or/and

the antigen-binding construct does not have significantly reduced binding affinity for ECD4 antigen or/and ECD2 antigen of HER2 compared to antigen-binding constructs in which the amino acid at position 30 is not E or/and the amino acid at position 53 is not Y

Embodiment 21. The antigen-binding construct according to any one of Embodiments 1-20, wherein the antigen-binding construct is afucosylated.

Embodiment 22. A pharmaceutical composition, comprising the antigen-binding construct according to any one of Embodiments 1-21 and a pharmaceutically acceptable carrier.

Embodiment 23. An isolated nucleic acid or a collection of isolated nucleic acids, comprising at least one nucleic acid sequence encoding at least one antigen-binding fragment of the antigen-binding construct according to any one of Embodiments 1-21.

Embodiment 24. A vector or a collection of vectors, comprising one or more of the nucleic acid and the collection of nucleic acids according to Embodiment 23.

Embodiment 25. An isolated cell, comprising the nucleic acid or the collection of nucleic acids according to Embodiment 23, or the vector or the collection of vectors according to claim 24.

Embodiment 26. A method for preparing the antigen-binding construct according to any one of Embodiments 1-21, comprising: culturing a host cell under conditions suitable for expression of the antigen-binding construct, wherein the host cell comprises a nucleic acid encoding the antigen-binding construct, or the vector or the collection of vectors according to Embodiment 24; and purifying the construct.

Embodiment 27. A method for treating a subject having an HER2-expressing tumor, comprising administering to the subject a therapeutically effective amount of the antigen-binding construct according to any one of Embodiments 1-21 or the pharmaceutical composition according to Embodiment 22.

Embodiment 28. The method according to Embodiment 27, comprising contacting a tumor cell with the antigen-binding construct so that an HER2-expressing tumor cell is killed or growth of an HER2-expressing tumor cell is inhibited.

Embodiment 29. The method according to Embodiment 27 or 28, wherein the tumor is biliary tract cancer, carcinosarcoma, esophageal cancer, gastroesophageal junction cancer, breast cancer, gastric cancer, pancreatic cancer, head and neck cancer, colorectal cancer, renal cancer, cervical cancer, ovarian cancer, endometrial cancer, uterine cancer, malignant melanoma, pharyngeal cancer, oral cancer, or skin cancer.

Embodiment 30. The method according to any one of Embodiments 27-29, comprising administering to a cell an effective amount of the antigen-binding construct or the pharmaceutical composition so that HER2 signaling in the cell is inhibited, reduced or blocked.

Embodiment 31. A method for enhancing resistance of an antigen-binding construct to aggregation, wherein the antigen-binding construct comprises a first antigen-binding fragment that is monovalent and specifically binds to ECD4 antigen of HER2 on an HER2-expressing cell, the first antigen-binding fragment being an scFv; the method comprises modifying the antigen-binding construct so that a heavy chain variable region of the first antigen-binding fragment comprises an amino acid mutation at position 30 according to the Kabat numbering system and the amino acid is mutated into E, or/and that a light chain variable region of the first antigen-binding fragment comprises an amino acid mutation at position 53 according to the Kabat numbering system and the amino acid is mutated into Y

Embodiment 32. The method for enhancing resistance of an antigen-binding construct to aggregation according to Embodiment 31, wherein the antigen-binding construct further comprises a second antigen-binding fragment that is monovalent and specifically binds to ECD2 antigen of HER2 on an HER2-expressing cell, and the second antigen-binding fragment is an Fab.

Embodiment 33. The method for enhancing resistance of an antigen-binding construct to aggregation according to Embodiment 31 or 32, wherein the first antigen-binding fragment comprises a group of CDRs selected from:

i. a group comprising a heavy chain CDR1, a heavy chain CDR2 and a heavy chain CDR3, wherein the heavy chain CDR1, the heavy chain CDR2 and the heavy chain CDR3 comprise the amino acid sequences according to SEQ ID NOs: 27, 28 and 29, respectively;

ii. a group comprising a heavy chain CDR1, a heavy chain CDR2 and a heavy chain CDR3, wherein the heavy chain CDR1, the heavy chain CDR2 and the heavy chain CDR3 comprise amino acid sequences having at least 80% identity to the amino acid sequences according to SEQ ID NOs: 27, 28 and 29, respectively;

iii. a group comprising a light chain CDR1, a light chain CDR2 and a light chain CDR3, wherein the light chain CDR1, the light chain CDR2 and the light chain CDR3 comprise the amino acid sequences according to SEQ ID NOs: 30, 34 and 32, respectively;

iv. a group comprising a light chain CDR1, a light chain CDR2 and a light chain CDR3, wherein the light chain CDR1, the light chain CDR2 and the light chain CDR3 comprise amino acid sequences having at least 80% identity to the amino acid sequences according to SEQ ID NOs: 30, 34 and 32, respectively;

v. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 27, 28 and 29, respectively; and the light chain CDR1, the light chain CDR2 and the light chain CDR3 comprise the amino acid sequences according to SEQ ID NOs: 30, 34 and 32, respectively;

vi. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise amino acid sequences having at least 80% identity to the amino acid sequences according to SEQ ID NOs: 27, 28 and 29, respectively; and the light chain CDR1, the light chain CDR2 and the light chain CDR3 comprise amino acid sequences having at least 80% identity to the amino acid sequences according to SEQ ID NOs: 30, 34 and 32, respectively;

vii. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 43, 28 and 29, respectively; and the light chain CDR1, the light chain CDR2 and the light chain CDR3 comprise the amino acid sequences according to SEQ ID NOs: 30, 31 and 32, respectively;

viii. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 43, 28 and 29, respectively; and the light chain CDR1, the light chain CDR2 and the light chain CDR3 comprise the amino acid sequences according to SEQ ID NOs: 30, 33 and 32, respectively;

ix. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 44, 28 and 29, respectively; and the light chain CDR1, the light chain CDR2 and the light chain CDR3 comprise the amino acid sequences according to SEQ ID NOs: 30, 31 and 32, respectively; and

ix. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 44, 28 and 29, respectively; and the light chain CDR1, the light chain CDR2 and the light chain CDR3 comprise the amino acid sequences according to SEQ ID NOs: 30, 31 and 32, respectively.

Embodiment 34. The method for enhancing resistance of an antigen-binding construct to aggregation according to Embodiment 31 or 32, wherein the first antigen-binding fragment is an scFv and comprises a heavy chain variable region of trastuzumab or a mutant thereof and a light chain variable region of trastuzumab or a mutant thereof, so that the mutant of the heavy chain variable region comprises a K30E mutation and the mutant of the light chain variable region comprises an F53Y mutation.

**Example 1. Construction, Expression and Purification of Anti-Her2-scFv-Fc and Variants Thereof**

In constructing anti-Her2-scFv-Fc, human IgG1 was used as the Fc portion, and the variable region sequence of the anti-Her2 arm was a sequence based on the monoclonal antibody Herceptin®. The light and heavy chain variable regions of the monoclonal antibody Herceptin® were linked in series by a designed linker 1 ((GGGGS)3, SEQ ID NO: 53) to form anti-Her2-scFv-Fc (SEQ ID NO: 1), the amino acid sequence of which is as follows:

EVQLVESGGGLVQPGGSLRLSCAASGFNIKDTYIHWVRQAPGKGLEWVARIYPTNGYTRYADSVKGRFTI
SADTSKNTAYLQMNSLRAEDTAVYYCSRWGGDGFYAMDYWGQGTLVTVSSGGGGSGGGGSGGGGSDI
QMTQSPSSLSASVGDRVTITCRASQDVNTAVAWYQQKPGKAPKLLIYSASFLYSGVPSRFSGSRSGTDFTL
TISSLQPEDFATYYCQQHYTTPPTFGQGTKVEIKGEPKSSDKTHTCPPCPAPELLGGPSVFLFPPKPKDTLMI
SRTPEVTCVVVDVSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK.

Further, point mutations were constructed in the anti-Her2-scFv-Fc sequence to construct the following variants:

anti-Her2-scFv-VL-F53Y-Fc (SEQ ID NO: 3): derived from wild-type anti-Her2-scFv-Fc, with F53Y in the VL region; the amino acid sequence is as follows:

EVQLVESGGGLVQPGGSLRLSCAASGFNIKDTYIHWVRQAPGKGLEWVARIYPTNGYTRYADSVKGRFTI
SADTSKNTAYLQMNSLRAEDTAVYYCSRWGGDGFYAMDYWGQGTLVTVSSGGGGSGGGGSGGGGSDI
QMTQSPSSLSASVGDRVTITCRASQDVNTAVAWYQQKPGKAPKLLIYSASYLYSGVPSRFSGSRSGTDFTL
TISSLQPEDFATYYCQQHYTTPPTFGQGTKVEIKGEPKSSDKTHTCPPCPAPELLGGPSVFLFPPKPKDTLMI
SRTPEVTCVVVDVSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK.

anti-Her2-scFv-VL-F53A-Fc (SEQ ID NO: 5): derived from wild-type anti-Her2-scFv-Fc, with F53A in the VL region; the amino acid sequence is as follows:

EVQLVESGGGLVQPGGSLRLSCAASGFNIKDTYIHWVRQAPGKGLEWVARIYPTNGYTRYADSVKGRFTI
SADTSKNTAYLQMNSLRAEDTAVYYCSRWGGDGFYAMDYWGQGTLVTVSSGGGGSGGGGSGGGGSDI
QMTQSPSSLSASVGDRVTITCRASQDVNTAVAWYQQKPGKAPKLLIYSASALYSGVPSRFSGSRSGTDFTL
TISSLQPEDFATYYCQQHYTTPPTFGQGTKVEIKGEPKSSDKTHTCPPCPAPELLGGPSVFLFPPKPKDTLMI
SRTPEVTCVVVDVSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK.

anti-Her2-scFv-VL-F53R-Fc (SEQ ID NO: 7): derived from wild-type anti-Her2-scFv-Fc, with F53R in the VL region; the amino acid sequence is as follows:

EVQLVESGGGLVQPGGSLRLSCAASGFNIKDTYIHWVRQAPGKGLEWVARIYPTNGYTRYADSVKGRFTI
SADTSKNTAYLQMNSLRAEDTAVYYCSRWGGDGFYAMDYWGQGTLVTVSSGGGGSGGGGSGGGGSDI
QMTQSPSSLSASVGDRVTITCRASQDVNTAVAWYQQKPGKAPKLLIYSASRLYSGVPSRFSGSRSGTDFTL
TISSLQPEDFATYYCQQHYTTPPTFGQGTKVEIKGEPKSSDKTHTCPPCPAPELLGGPSVFLFPPKPKDTLMI
SRTPEVTCVVVDVSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK.

anti-Her2-scFv-VH-K30E-Fc (SEQ ID NO: 9): derived from wild-type anti-Her2-scFv-Fc, with K30E in the VH region; the amino acid sequence is as follows:

EVQLVESGGGLVQPGGSLRLSCAASGFNIEDTYIHWVRQAPGKGLEWVARIYPTNGYTRYADSVKGRFTI
SADTSKNTAYLQMNSLRAEDTAVYYCSRWGGDGFYAMDYWGQGTLVTVSSGGGGSGGGGSGGGGSDI
QMTQSPSSLSASVGDRVTITCRASQDVNTAVAWYQQKPGKAPKLLIYSASFLYSGVPSRFSGSRSGTDFTL
TISSLQPEDFATYYCQQHYTTPPTFGQGTKVEIKGEPKSSDKTHTCPPCPAPELLGGPSVFLFPPKPKDTLMI
SRTPEVTCVVVDVSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK.

The DNA sequences of anti-Her2-scFv-Fc and the variants thereof (SEQ ID NOs: 2, 4, 6, 8 and 10) were synthesized and each cloned into the pcDNA3.1 expression vector. The expression vector of anti-Her2-scFv-Fc or the variants thereof was transfected into ExpiCHO cells (CHO-S, Thermo) by using an ExpiCHO™ expression kit (Thermo Fisher, Cat. No. A29133). The cells were cultured in ExpiCHO expression medium in a humidified atmosphere in an incubator at 37 °C with 8% $CO_2$ on an orbital shaker spinning at 130 rpm. The culture supernatant was collected, and protein purification was performed using protein A magnetic beads (Genscript, Cat. No. L00273). The protein concentration was measured using a UV-Vis spectrophotometer (NanoDrop lite, Thermo Scientific).

Table 1: Sequence information about anti-Her2-scFv-Fc and the variants thereof

| Name | Amino acid sequence (SEQ ID NO) | Encoding DNA sequence (SEQ ID NO) | Mutation site in variable region |
|---|---|---|---|
| anti-Her2-scFv-Fc | 1 | 2 | None |
| anti-Her2-scFv-VL-F53Y-Fc | 3 | 4 | F53Y |
| anti-Her2-scFv-VL-F53A-Fc | 5 | 6 | F53A |
| anti-Her2-scFv-VL-F53R-Fc | 7 | 8 | F53R |
| anti-Her2-scFv-VH-K30E-Fc | 9 | 10 | K30E |

**Example 2. Aggregate Verification and Human Her2 Antigen-Binding Assay of Anti-Her2-scFv-Fc and Variants Thereof**

For the expressed and purified anti-Her2-scFv-Fc and variants thereof, the affinity of the test molecules for human Her2 protein was determined by Biacore T200 (GE) as follows:

An amount of anti-Her2-scFv-Fc or a variant thereof was captured by a chip coupled to Anti-hIgG, and then an antigen human HER2 protein (Sino Biological, Cat. No. 10004-H08H) was allowed to flow over the chip surface.

The response signals were detected in real time using Biacore T200, and association and dissociation curves were obtained. The buffer used in the experiment was Biacore universal buffer (137 mM NaCl, 2.7 mM KCl, 10 mM $Na_2HPO_4 \cdot 12H_2O$, 1.8 mM $KH_2PO_4$, 0.05% surfactant P20 (GE, Cat. No. BR-1000-54), pH 7.4). Anti-hIgG (from human antibody capture kit, GE, Cat. No. 29-2346-00) was coupled to a CM5 chip surface at a response value of up to about 9000 RU, and anti-Her2-scFv-Fc or a variant thereof was captured at about 200 RU. Then the signal values of the interaction of different concentrations of human HER2 protein (100 nM, 50 nM, 25 nM, 12.5 nM, 6.25 nM and 3.125 nM) with anti-Her2-scFv-Fc or the variant thereof were measured. The flow rate in the flow cell was at 50 μL/min, the association was performed for 240 s, the dissociation was performed for 1400 s, the regeneration was performed using 3 M $MgCl_2$ (GE) for 60 s, and the baseline was stable. Results were obtained by calculation according to the affinity and kinetics 1:1 binding mode in biacore evaluation software. The affinity of anti-Her2-scFv-Fc or the variants for the antigen human Her2 protein is shown in Table 2. Compared to anti-Her2-scFv-Fc (KD = 0.77 nM), the variant anti-Her2-scFv-VL-F53A-Fc (KD = 1.26 nM) had a greater effect on the binding affinity for the antigen human Her2 protein. The variant anti-Her2-scFv-VL-F53Y-Fc (KD = 0.8 nM) and anti-Her2-scFv-Fc (KD = 0.77 nM) showed similar binding affinity for the antigen human Her2 protein. The variant anti-Her2-scFv-VH-K30E-Fc has a binding KD of 0.54 nM for the antigen human Her2 protein. It is known that the introduction of mutations F53Y and K30E does not reduce the binding affinity for the antigen human Her2 protein.

Components of anti-Her2-scFv-Fc or the variants thereof were separated by gel column chromatography. Elution was performed using a neutral buffer as the mobile phase, and the components with different molecular weights were eluted out in descending order according to their molecular weights. The gel chromatography column used was an ACQUITY UPLC Protein BEH SEC Column 200 Å, 1.7 μm, 4.6 × 300 mm, and the column temperature was at 25 °C. The mobile phase was 50 mmol/L phosphate-buffered saline-200 mmol/L sodium chloride at pH 7.0 (2.33 g of sodium dihydrogen phosphate dihydrate, 12.53 g of sodium dihydrogen phosphate dodecahydrate and 11.69 g of sodium chloride were weighed into about 800 mL of ultrapure water and completely dissolved by stirring; ultrapure water was added until the volume reached 1000 mL; the mixture was well mixed and then filtered through a 0.22 μm filter membrane). The sample was diluted with the mobile phase to obtain a 10 mg/mL test solution, 2 μL of which was precisely measured out and injected into a liquid chromatograph (adjusting the volume of injection so that 20 μg of protein was injected if the sample concentration was less than 10 mg/mL) for detected at 280 nm.

The flow rate was at 0.30 mL/min, and isocratic elution was performed for 15 min. Data was processed, and the results were quantitatively analyzed using the area normalization method. The peak area percentages for the aggregate, immunoglobulin monomer and low molecular weight impurities were calculated respectively. The aggregate peaks appeared before the main peak, which represents the immunoglobulin monomer, and the low molecular weight impurity peaks appeared after the main peak. The main peak and aggregate content percentages in anti-Her2-scFv-Fc or the variants thereof are shown in Table 2. The variants anti-Her2-scFv-VL-F53Y-Fc and anti-Her2-scFv-VH-K30E-Fc have significantly reduced aggregate: the aggregate content was reduced from 6.31% (in anti-Her2-scFv-Fc) to 4.94% and 3.39%, respectively.

Table 2: Aggregate content and binding affinity of anti-Her2-scFv-Fc and variants thereof for antigen Her2

| Name | anti-Her2-scFv-Fc | anti-Her2-scFv-VL-F53Y-Fc | anti-Her2-scFv-VL-F53A-Fc | anti-Her2-scFv-VL-F53R-Fc | anti-Her2-scFv-VH-K30E-Fc |
|---|---|---|---|---|---|
| Aggregate | 6.31% | 4.94% | 6.96% | 7.83% | 3.39% |
| Monomer | 93.43% | 94.83% | 92.80% | 91.90% | 96.32% |
| Low molecular weight fragments | 0.25% | 0.24% | 0.23% | 0.26% | 0.28% |
| Antigen Her2 (KD) | 0.77 nM | 0.8 nM | 1.26 nM | Not measured | 0.54 nM |

**Example 3. Construction, Expression and Purification of Anti-Her2 Bispecific Antibodies**

Anti-Her2 bispecific antibodies were produced as human IgG1 by knobs-into-holes (Ridgway, et al., 1996) Fc engineering. H435R and Y436F mutations (Jendeberg et al.,1997) were designed in the Fc region sequence of one heavy chain to reduce the affinity of Fc for protein A, which was favorable for removing the homodimers formed during the assembly of bispecific antibodies in the protein A affinity purification (Patent US5945311A). It can be seen from Example 2 that anti-Her2-scFv-VL-F53Y-Fc mutation and anti-Her2-scFv-VH-K30E-Fc mutation could significantly reduce the anti-Her2-scFv aggregate while not reduce the affinity for Her2 antigen. The antigen-binding domain of one anti-Her2 arm of the anti-Her2 bispecific antibodies in this example was in scFv (VH-linker-VL structure) form, and the variable region sequences comprised a mutation K30E (b-anti-Her2-scFv-VH-K30E-Fc, SEQ ID NO: 21), a mutation F53Y (b-anti-Her2-scFv-VL-F53Y-Fc, SEQ ID NO: 23), or both of those point mutations (anti-Her2-scFv-VH-K30E-VL-F53Y-Fc, SEQ ID NO: 11). The antigen-binding domain of another anti-Her2 arm of the anti-Her2 bispecific antibodies in this example was in Fab form, including anti-Her2-domain2-HC-Fc (SEQ ID NO: 13) and anti-Her2-domain2-LC (SEQ ID NO: 15). Additionally, an anti-Her2 bispecific antibody was constructed using scFv (VH-linker-VL structure or VL-linker-VH structure) without mutations as a control.

Table 3: Sequence information about anti-Her2 bispecific antibodies

| Name | Composition | Amino acid sequence (SEQ ID NO) | Nucleotide sequence (SEQ ID NO) |
|---|---|---|---|
| Expi Her2-1 | anti-Her2-scFv-VL-VH-Fc | DIQMTQSPSSLSASVGDRVTITCRASQDVNTAVAW YQQKPGKAPKLLIYSASFLYSGVPSRFSGSRSGTDF TLTISSLQPEDFATYYCQQHYTTPPTFGQGTKVEIK GGSGGGSGGGSGGGSGGGSGEVQLVESGGGLVQP GGSLRLSCAASGFNIKDTYIHWVRQAPGKGLEWV ARIYPTNGYTRYADSVKGRFTISADTSKNTAYLQM NSLRAEDTAVYYCSRWGGDGFYAMDYWGQGTL VTVSSAAEPKSSDKTHTCPPCPAPELLGGPSVFLFP PKPKDTLMISRTPEVTCVVVDVSHEDPEVKFNWY VDGVEVHNAKTKPREEQYNSTYRVVSVLTVLHQD WLNGKEYKCKVSNKALPAPIEKTISKAKGQPREPQ VYTLPPSRDELTKNQVSLICLVKGFYPSDIAVEWES NGQPENRYMTWPPVLDSDGSFFLYSKLTVDKSRW QQGNVFSCSVMHEALHNHYTQKSLSLSPGK (SEQ ID NO: 17) | 18 |
| | anti-Her2-domain2-HC-Fc-2 | EVQLVESGGGLVQPGGSLRLSCAASGFTFTDYTM DWVRQAPGKGLEWVADVNPNSGGSIYNQRFKGR FTLSVDRSKNTLYLQMNSLRAEDTAVYYCARNLG PSFYFDYWGQGTLVTVSSASTKGPSVFPLAPSSKST SGGTAALGCLVKDYFPEPVTVSWNSGALTSGVHT FPAVLQSSGLYSLSSVVTVPSSSLGTQTYICNVNHK PSNTKVDKKVEPKSCDKTHTCPPCPAPELLGGPSV FLFPPKPKDTLMISRTPEVTCVVVDVSHEDPEVKFN WYVDGVEVHNAKTKPREEQYNSTYRVVSVLTVL HQDWLNGKEYKCKVSNKALPAPIEKTISKAKGQP REPQVYVYPPSRDELTKNQVSLTCLVKGFYPSDIA VEWESNGQPENNYKTTPPVLDSDGSFALVSKLTV DKSRWQQGNVFSCSVMHEALHNRFTQKSLSLSPG (SEQ ID NO: 19) | 20 |
| | anti-Her2-domain2-LC | DIQMTQSPSSLSASVGDRVTITCKASQDVSIGVAW YQQKPGKAPKLLIYSASYRYTGVPSRFSGSGSGTD FTLTISSLQPEDFATYYCQQYYIYPYTFGQGTKVEI KRTVAAPSVFIFPPSDEQLKSGTASVVCLLNNFYPR EAKVQWKVDNALQSGNSQESVTEQDSKDSTYSLS STLTLSKADYEKHKVYACEVTHQGLSSPVTKSFNR GEC (SEQ ID NO: 15) | 16 |

(continued)

| Name | Composition | Amino acid sequence (SEQ ID NO) | Nucleotide sequence (SEQ ID NO) |
|---|---|---|---|
| Expi Her2-2 | anti-Her2-scFv-VH-K30E-VL-F53Y-Fc | EVQLVESGGGLVQPGGSLRLSCAASGFNIEDTYIH WVRQAPGKGLEWVARIYPTNGYTRYADSVKGRF TISADTSKNTAYLQMNSLRAEDTAVYYCSRWGGD GFYAMDYWGQGTLVTVSSGGGGSGGGGSGGGGS GGGGSDIQMTQSPSSLSASVGDRVTITCRASQDVN TAVAWYQQKPGKAPKLLIYSASYLYSGVPSRFSGS RSGTDFTLTISSLQPEDFATYYCQQHYTTPPTFGQG TKVEIKGEPKSSDKTHTCPPCPAPELLGGPSVFLFPP KPKDTLMISRTPEVTCVVVDVSHEDPEVKFNWYV DGVEVHNAKTKPREEQYNSTYRVVSVLTVLHQD WLNGKEYKCKVSNKALPAPIEKTISKAKGQPREPQ VYTLPPCREEMTKNQVSLWCLVKGFYPSDIAVEW ESNGQPENNYKTTPPVLDSDGSFFLYSKLTVDKSR WQQGNVFSCSVMHEALHNHYTQKSLSLSPGK (SEQ ID NO: 11) | 12 |
| | anti-Her2-domain2-HC-Fc | EVQLVESGGGLVQPGGSLRLSCAASGFTFTDYTM DWVRQAPGKGLEWVADVNPNSGGSIYNQRFKGR FTLSVDRSKNTLYLQMNSLRAEDTAVYYCARNLG PSFYFDYWGQGTLVTVSSASTKGPSVFPLAPSSKST SGGTAALGCLVKDYFPEPVTVSWNSGALTSGVHT FPAVLQSSGLYSLSSVVTVPSSSLGTQTYICNVNHK PSNTKVDKKVEPKSCDKTHTCPPCPAPELLGGPSV FLFPPKPKDTLMISRTPEVTCVVVDVSHEDPEVKFN WYVDGVEVHNAKTKPREEQYNSTYRVVSVLTVL HQDWLNGKEYKCKVSNKALPAPIEKTISKAKGQP REPQVCTLPPSREEMTKNQVSLSCAVKGFYPSDIA VEWESNGQPENNYKTTPPVLDSDGSFFLVSKLTVD KSRWQQGNVFSCSVMHEALHNRFTQKSLSLSPGK (SEQ ID NO: 13) | 14 |
| | anti-Her2-domain2-LC | DIQMTQSPSSLSASVGDRVTITCKASQDVSIGVAW YQQKPGKAPKLLIYSASYRYTGVPSRFSGSGSGTD FTLTISSLQPEDFATYYCQQYYIYPYTFGQGTKVEI KRTVAAPSVFIFPPSDEQLKSGTASVVCLLNNFYPR EAKVQWKVDNALQSGNSQESVTEQDSKDSTYSLS STLTLSKADYEKHKVYACEVTHQGLSSPVTKSFNR GEC (SEQ ID NO: 15) | 16 |

(continued)

| Name | Composition | Amino acid sequence (SEQ ID NO) | Nucleotide sequence (SEQ ID NO) |
|---|---|---|---|
| Expi Her2-3 | b-anti-Her2-scFv-VH-K30E-Fc | EVQLVESGGGLVQPGGSLRLSCAASGFNIEDTYIH WVRQAPGKGLEWVARIYPTNGYTRYADSVKGRF TISADTSKNTAYLQMNSLRAEDTAVYYCSRWGGD GFYAMDYWGQGTLVTVSSGGGGSGGGGSGGGGS GGGGSDIQMTQSPSSLSASVGDRVTITCRASQDVN TAVAWYQQKPGKAPKLLIYSASFLYSGVPSRFSGS RSGTDFTLTISSLQPEDFATYYCQQHYTTPPTFGQG TKVEIKGEPKSSDKTHTCPPCPAPELLGGPSVFLFPP KPKDTLMISRTPEVTCVVVDVSHEDPEVKFNWYV DGVEVHNAKTKPREEQYNSTYRVVSVLTVLHQD WLNGKEYKCKVSNKALPAPIEKTISKAKGQPREPQ VYTLPPCREEMTKNQVSLWCLVKGFYPSDIAVEW ESNGQPENNYKTTPPVLDSDGSFFLYSKLTVDKSR WQQGNVFSCSVMHEALHNHYTQKSLSLSPGK (SEQ ID NO: 21) | 22 |
| | anti-Her2-domain2-HC-Fc | SEQ ID NO: 13 | 14 |
| | anti-Her2-domain2-LC | SEQ ID NO: 15 | 16 |
| Expi Her2-4 | b-anti-Her2-scFv-VL-F53Y-Fc | EVQLVESGGGLVQPGGSLRLSCAASGFNIKDTYIH WVRQAPGKGLEWVARIYPTNGYTRYADSVKGRF TISADTSKNTAYLQMNSLRAEDTAVYYCSRWGGD GFYAMDYWGQGTLVTVSSGGGGSGGGGSGGGGS GGGGSDIQMTQSPSSLSASVGDRVTITCRASQDVN TAVAWYQQKPGKAPKLLIYSASYLYSGVPSRFSGS RSGTDFTLTISSLQPEDFATYYCQQHYTTPPTFGQG TKVEIKGEPKSSDKTHTCPPCPAPELLGGPSVFLFPP KPKDTLMISRTPEVTCVVVDVSHEDPEVKFNWYV DGVEVHNAKTKPREEQYNSTYRVVSVLTVLHQD WLNGKEYKCKVSNKALPAPIEKTISKAKGQPREPQ VYTLPPCREEMTKNQVSLWCLVKGFYPSDIAVEW ESNGQPENNYKTTPPVLDSDGSFFLYSKLTVDKSR WQQGNVFSCSVMHEALHNHYTQKSLSLSPGK (SEQ ID NO: 23) | 24 |
| | anti-Her2-domain2-HC-Fc | SEQ ID NO: 13 | 14 |
| | anti-Her2-domain2-LC | SEQ ID NO: 15 | 16 |

(continued)

| Name | Composition | Amino acid sequence (SEQ ID NO) | Nucleotide sequence (SEQ ID NO) |
|---|---|---|---|
| Expi Her2-5 | anti-Her2-scFv-VH-VL-Fc | EVQLVESGGGLVQPGGSLRLSCAASGFNIKDTYIH WVRQAPGKGLEWVARIYPTNGYTRYADSVKGRF TISADTSKNTAYLQMNSLRAEDTAVYYCSRWGGD GFYAMDYWGQGTLVTVSSGGGGSGGGGSGGGGS GGGGSDIQMTQSPSSLSASVGDRVTITCRASQDVN TAVAWYQQKPGKAPKLLIYSASFLYSGVPSRFSGS RSGTDFTLTISSLQPEDFATYYCQQHYTTPPTFGQG TKVEIKGEPKSSDKTHTCPPCPAPELLGGPSVFLFPP KPKDTLMISRTPEVTCVVVDVSHEDPEVKFNWYV DGVEVHNAKTKPREEQYNSTYRVVSVLTVLHQD WLNGKEYKCKVSNKALPAPIEKTISKAKGQPREPQ VYTLPPCREEMTKNQVSLWCLVKGFYPSDIAVEW ESNGQPENNYKTTPPVLDSDGSFFLYSKLTVDKSR WQQGNVFSCSVMHEALHNHYTQKSLSLSPGK (SEQ ID NO: 25) | 26 |
| | anti-Her2-domain2-HC-Fc | SEQ ID NO: 13 | 14 |
| | anti-Her2-domain2-LC | SEQ ID NO: 15 | 16 |

The DNA sequences of anti-Her2 bispecific antibodies (SEQ ID NOs: 12, 14 and 16) were synthesized and each cloned into the pcDNA3.1 expression vector. The expression vectors of anti-Her2-scFv-VH-K30E-VL-F53Y-Fc (SEQ ID NO: 11), anti-Her2-domain2-HC-Fc (SEQ ID NO: 13) and anti-Her2-domain2-LC (SEQ ID NO: 15) were co-transfected into ExpiCHO cells in a transfection ratio of 1:1:1.5 using an ExpiCHO™ expression kit (Thermo Fisher, Cat. No. A29133). The transfection density was $6 \times 10^6$ cells/mL. The medium was an ExpiCHO expression medium. The culture was continued until day 10 after transfection, and the cell culture supernatant was collected by centrifugation. Protein purification was performed using protein A magnetic beads (Genscript, Cat. No. L00273). The protein concentration was measured using a UV-Vis spectrophotometer (NanoDrop lite, Thermo Scientific). This sample was designated Expi Her2-2. With reference to this method, other bispecific antibodies, Expi Her2-1, Expi Her2-3, Expi Her2-4 and Expi Her2-5, were obtained by expression and purification according to the DNA sequences given in the table above.

**Example 4. Preparation and Verification of De-fucosylated Bispecific Antibodies**

The interaction of IgG1 with FcgRIIIa can be improved by knocking out the fucose expression-related gene FUT8, and thereby the ADCC of the antibody is enhanced (Shields et al., 2002; Yamane-Ohnuki et al., 2004). In this example, fucose knockout anti-Her2 bispecific antibodies were prepared using FUT8- knockout CHO-S cells (designated CHO FUT8-/- cells). The DNA sequences of anti-Her2 bispecific antibodies (SEQ ID NOs: 12, 14 and 16) were synthesized and each cloned into the pcDNA3.1 expression vector. The expression vectors of anti-Her2-scFv-VH-K30E-VL-F53Y-Fc, anti-Her2-domain2-HC-Fc and anti-Her2-domain2-LC were co-transfected into the FUT8- knockout CHO-S cells in a transfection ratio of 1:1:1.5 using a CHOgro® high yield expression system (Cat. No. MIR 6270). The transfection density was $6 \times 10^6$ cells/mL. The medium was CHOgro® expression medium (Cat. No. MIR 6200, manufacturer: Mirus). The culture was continued until day 10 after transfection, and the cell culture supernatant was collected by centrifugation. Protein purification was performed using protein A magnetic beads (Genscript, Cat. No. L00273). The protein concentration was measured using a UV-Vis spectrophotometer (NanoDrop lite, Thermo Scientific). This sample was designated 23C2 Her2-2. With reference to this method, the sequences of Expi Her2-1, Expi Her2-3, Expi Her2-4 and Expi Her2-5 listed in Table 3 were expressed in the CHO FUT8-/- cells to obtain corresponding fucose knockout anti-Her2 bispecific antibodies 23C2 Her2-1, 23C2 Her2-3, 23C2 Her2-4 and 23C2 Her2-5.

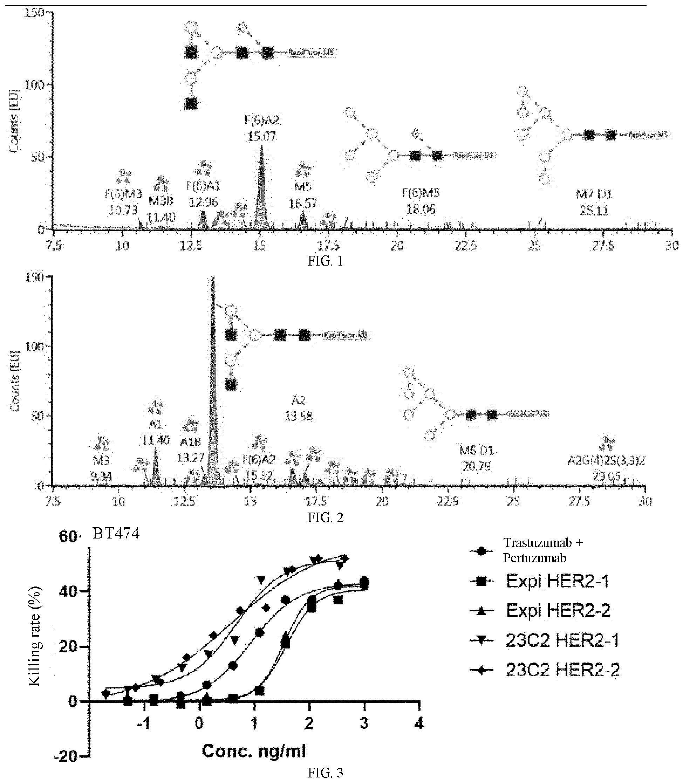

The anti-Her2 bispecific antibody samples expressed by the CHO FUT8-/- cells and CHO-S cells were processed using a GlycoWorks RapiFluor-MS N-Glycan kit (Waters, Milford, Mass., USA). The N-glycans were released from protein and labeled. After column chromatography separation, analysis was performed using an FLR detector (Waters, Milford, Mass., USA), and the structure and content of N-glycan could be obtained. The glycan mapping analysis of the anti-Her2 bispecific antibody Expi HER2-2 is shown in FIG. 1. The glycan mapping analysis of the anti-Her2 bispecific antibody 23C2 HER2-2 expressed by FUT8 knockout CHO-S cells is shown in FIG. 2. The glycan content percentages are shown in Table 4. In the normal anti-Her2 bispecific antibody Expi HER2-2, the percentage of de-fucosylated form was 21.85%, and in the anti-Her2 bispecific antibody 23C2 HER2-2 expressed by FUT8- knockout CHO-S cells, the percentage of de-fucosylated form was 99.40%.

Table 4: Glycoform content analysis of anti-Her2 bispecific antibodies

| Component name | Content (%) | |
| --- | --- | --- |
| | Expi HER2-2 | 23C2 HER2-2 |
| A1(M3B) | 1.81 | 9.84 |
| A1G(4)1 | 0.16 | 0.17 |
| A2 | 1.14 | 70.33 |
| A2[3]G(4)1 | / | 2.34 |
| A2[6]G(4)1 | / | 4.19 |
| A2[3]BG(4)1 | / | 0.11 |
| A2G(4)2 | / | 0.55 |
| A2G(4)2S(3)1 | / | 0.21 |
| A2G(4)2S(3,3)2 | / | 0.46 |
| F(6)A1G(4)1 | / | / |
| F(6)A1[3]G(4)1S(3)1 | / | / |
| F(6)A1 | 12.77 | / |
| F(6)A2 | 60.69 | 0.60 |
| F(6)A2[3]G(4)1 | 1.20 | / |
| F(6)A2[61G(4)1 | 0.70 | / |
| F(6)A2G(4)2 | / | / |
| F(6)A2G(4)2S(3)1 | / | / |
| M3 | / | 0.26 |
| M4 | / | 0.07 |
| M4A1G(4)1 | / | 0.36 |
| M4 D1 | / | 0.08 |
| M5 | 12.31 | 5.67 |
| M5A1G(4)1 | / | 0.05 |
| M6 D1 | 2.11 | 0.75 |
| M6 D3 | 0.73 | 0.18 |
| M7D1 | 0.80 | 0.44 |
| M8 | 0.20 | 0.15 |
| De-fucosylated glycans | 21.85 | 99.40 |
| Di-sialylated glycans | / | 0.46 |
| High mannose glycans | 20.07 | 7.24 |
| Mono-sialylated glycans | 0.18 | 0.21 |
| Non-sialylated glycans | 99.82 | 99.33 |

**Example 5. Aggregate Verification of Bispecific Antibodies**

This example relates to aggregate verification of anti-Her2 bispecific antibodies 23C2 Her2-1, 23C2 Her2-2, 23C2 Her2-3, 23C2 Her2-4 and 23C2 Her2-5.

The anti-Her2 bispecific antibodies were separated by gel column chromatography, and the aggregate content was verified. Elution was performed using a neutral buffer as the mobile phase, and the components with different molecular weights were eluted out in descending order according to their molecular weights. The gel chromatography column used was an ACQUITY UPLC Protein BEH SEC Column 200 Å, 1.7 $\mu$m, 4.6 × 300 mm, and the column temperature was at 25 °C. The mobile phase was 50 mmol/L phosphate-buffered saline-200 mmol/L sodium chloride at pH 7.0 (2.33 g of sodium dihydrogen phosphate dihydrate, 12.53 g of sodium dihydrogen phosphate dodecahydrate and 11.69 g of sodium chloride were weighed into about 800 mL of ultrapure water and completely dissolved by stirring; ultrapure water was added until the volume reached 1000 mL; the mixture was well mixed and then filtered through a 0.22 $\mu$m filter membrane). The sample was diluted with the mobile phase to obtain a 10 mg/mL test solution, 2 $\mu$L of which was precisely measured out and injected into a liquid chromatograph (adjusting the volume of injection so that 20 $\mu$g of protein was injected if the sample concentration was less than 10 mg/mL) for detected at 280 nm. The flow rate was at 0.30 mL/min, and isocratic elution was performed for 15 min. Data was processed, and the results were quantitatively analyzed using the area normalization method. The peak area percentages for the aggregate, immunoglobulin monomer and low molecular weight impurities were calculated respectively. The aggregate peaks appeared before the main peak, which represents the immunoglobulin monomer, and the low molecular weight impurity peaks appeared after the main peak.

Table 5: Aggregate content of anti-Her2 bispecific antibodies

| Sample name | Aggregate | Monomer | Low molecular weight fragments |
|---|---|---|---|
| 23C2 Her2-2 | 8.51% | 91.02% | 0.47% |
| 23C2 Her2-1 | 19.55% | 80.05% | 0.40% |

The results show that after scFVs (without mutations) were assembled into bispecific antibodies, the bispecific antibodies still produced a large amount of aggregate; however, the aggregate content in the bispecific antibodies can be reduced by introducing mutations. The aggregate content in 23C2 Her2-2 can be reduced to 8.51% compared to that in 23C2 Her2-1 comprising no mutation (Table 5).

**Example 6. Antigen-Binding Assay of Anti-Her2 Bispecific Antibodies**

For the expressed and purified anti-Her2 bispecific antibodies, the affinity of the test molecules for HER2 protein was determined by Biacore T200 (GE) as follows:
An amount of an anti-Her2 bispecific antibody was captured by a chip coupled to Anti-hIgG, and then human Her2 (Sino Biological, Cat. No. 10004-H08H) was allowed to flow over the chip surface. The response signals were detected in real time using Biacore T200, and association and dissociation curves were obtained. The buffer used in the experiment was Biacore universal buffer (137 mM NaCl, 2.7 mM KCl, 10 mM $Na_2HPO_4 \cdot 12H_2O$, 1.8 mM $KH_2PO_4$, 0.05% surfactant P20, pH 7.4). Anti-hIgG (from human antibody capture kit, GE, Cat. No. 29-2346-00) was coupled to a CM5 chip surface at a response value of up to about 9000 RU, and the anti-Her2 bispecific antibody was captured at about 200 RU. Then the signal values of the interaction of different concentrations of Her2 protein (100 nM, 50 nM, 25 nM, 12.5 nM, 6.25 nM and 3.125 nM) with the anti-Her2 bispecific antibody were measured.

The flow rate in the flow cell was at 50 $\mu$L/min, the association was performed for 240 s, the dissociation was performed for 1400 s, the regeneration was performed using 3 M $MgCl_2$ (GE) for 60 s, and the baseline was stable.

The results were obtained by calculation using biacore evaluation software. The binding affinity of the anti-Her2 bispecific antibody 23C2 Her2-2, as well as the controls trastuzumab and pertuzumab, for antigen Her2 is shown in Table 6. The binding KD of the anti-Her2 bispecific antibody 23C2 Her2-2 for antigen Her2 is 6.11E-10 M, the binding KD of trastuzumab for antigen Her2 is 1.22E-09 M, and the binding KD of pertuzumab for antigen Her2 is 2.35E-09 M. The anti-Her2 bispecific antibody 23C2 Her2-2 showed higher affinity than trastuzumab and pertuzumab for antigen Her2.

Table 6: Affinity of anti-Her2 bispecific antibodies for human Her2 antigen

| | Ka (1/Ms) | Kd (1/s) | KD (M) |
|---|---|---|---|
| 23C2 Her2-2 | 1.79E+05 | 1.09E-04 | 6.11E-10 |

(continued)

|  | Ka (1/Ms) | Kd (1/s) | KD (M) |
|---|---|---|---|
| Trastuzumab control | 1.73E+05 | 2.12E-04 | 1.22E-09 |
| Pertuzumab control | 1.15E+05 | 2.69E-04 | 2.35E-09 |

## Example 7. Antigen-Binding Assay of Anti-Her2 Bispecific Antibodies

With reference to the procedures in Example 6, the affinity of the expressed and purified anti-Her2 bispecific antibodies 23C2 Her2-1, 23C2 Her2-2, 23C2 Her2-3, 23C2 Her2-4 and 23C2 Her2-5 for HER2 protein was measured by Biacore T200 (GE).

The affinity of the anti-Her2 bispecific antibodies for the antigen human Her2 protein is shown in Table 7. It can be seen from the results in Tables 6 and 7 that the binding affinity of the anti-Her2 bispecific antibodies for the antigen human Her2 protein can be improved by introducing F53Y and K30E mutations.

Table 7: Affinity of anti-Her2 bispecific antibodies for human Her2 antigen

| Sample name | 23C2 Her2-1 |
|---|---|
| Antigen Her2 KD | 4.02 nM |

## Example 8. Killing of Her2-Positive Target Cell BT474 by Anti-Her2 Bispecific Antibodies

The killing effects of the anti-Her2 bispecific antibodies on target cells (BT474 Her2+++, source: the Cell Bank of Type Culture Collection Committee of the Chinese Academy of Sciences) were studied using NK cells provided by human PBMCs (peripheral blood mononuclear cells; effector cells). $EC_{50}$ was calculated, and the *in vitro* activity of the anti-Her2 bispecific antibodies was assessed.

The specific procedures are as follows:

BT474 cells were adjusted to a cell density of $3 \times 10^5$ cells/mL using 1640 medium containing 2% FBS (fetal bovine serum) and seeded in a 96-well cell culture plate (eppendorf, Cat. No. 0030730199) at 50 $\mu$L per well. Different concentrations of the anti-Her2 bispecific antibodies (1000 ng/mL, 333 ng/mL, 111 ng/mL, 37 ng/mL, 12.3 ng/mL, 4.11 ng/mL, 1.37 ng/mL, 0.46 ng/mL, 0.15 ng/mL and 0.05 ng/mL) were prepared using 1640 medium and added to the above 96-well cell culture plate at 50 $\mu$L per well. Human PBMCs were adjusted to a cell density of $1.5 \times 10^6$ cells/mL using 1640 medium and added at 100 $\mu$L per well. An administration group (target cell + effector cell + antibody), a target cell group (BT474 cell), an effector cell group (human PBMC), a target cell + effector cell group, a blank control group (medium) and a lysis solution control group, and a target cell maximum release group (target cell + lysis solution) were set, with the effector-to-target cell ratio being 10:1. Forty-five minutes prior to the assay, 20 $\mu$L/well of lysis solution (Promega, Cat. No. G182A) was added to the target cell maximum release group and the lysis solution control group. After 45 min, the cell lysis rates (killing rates) were measured using a CytoTox96® nonradioactive cytotoxicity assay kit (Promega, G1780).

$$\text{Rate of lysis (\%)} = (\text{OD}_{\text{administration group}} - \text{OD}_{\text{target cell + effector cell group}})/(\text{OD}_{\text{target cell maximum release group}} - \text{OD}_{\text{target cell group}}) \times 100\%$$

FIG. 3 shows the killing rates of the anti-Her2 bispecific antibodies against BT474 Her2+++ tumor cells. The ADCC-enhanced anti-Her2 bispecific antibodies 23C2 HER2-1 and 23C2 HER2-2 had better killing effects on BT474 tumor cells than the combination of trastuzumab and pertuzumab and than the anti-Her2 bispecific antibodies Expi Her2-1 and Expi HER2-2 expressed by CHO-S cells, wherein the $EC_{50}$ of the combination of trastuzumab and pertuzumab (1:1) is 8.627 ng/mL; the $EC_{50}$ of Expi HER2-1 is 38.05 ng/mL; having an $EC_{50}$ of 35.17 ng/mL, the Expi HER2-2 is superior to Expi HER2-1; the $EC_{50}$ of the ADCC-enhanced 23C2 HER2-1 is 4.728 ng/mL; having an $EC_{50}$ of 3.658 ng/mL, the ADCC-enhanced 23C2 HER2-2 is superior to 23C2 HER2-1.

## Example 9. Killing of Her2-Positive Target Cell NCI-N87 by Anti-Her2 Bispecific Antibodies

The killing effects of the anti-Her2 bispecific antibodies on target cells (NCI-N87 Her2++, source: the Cell Bank of Type Culture Collection Committee of the Chinese Academy of Sciences) were studied using NK cells provided by human PBMCs (peripheral blood mononuclear cells; effector cells). $EC_{50}$ was calculated, and the *in vitro* activity of the anti-Her2 bispecific antibodies was assessed.

The specific procedures are as follows:

NCI-N87 cells were adjusted to a cell density of $3 \times 10^5$ cells/mL using 1640 medium containing 2% FBS (fetal bovine serum) and seeded in a 96-well cell culture plate (eppendorf, Cat. No. 0030730199) at 50 $\mu$L per well. Different concentrations of the anti-Her2 bispecific antibodies or control drugs (8.1 nM, 2.7 nM, 0.9 nM, 0.3 nM, 0.1 nM, 0.03 nM, 0.01 nM, 0.003 nM, 0.001 nM and 0.0004 nM) were prepared using 1640 medium and added to the above 96-well cell culture plate at 50 $\mu$L per well. Human PBMCs were adjusted to a cell density of $1.5 \times 10^6$ cells/mL using 1640 medium and added at 100 $\mu$L per well. An administration group (target cell + effector cell + antibody), a target cell group (NCI-N87 cell), an effector cell group (human PBMC), a target cell + effector cell group, a blank control group (medium) and a lysis solution control group, and a target cell maximum release group (target cell + lysis solution) were set, with the effector-to-target cell ratio being 10:1. Forty-five minutes prior to the assay, 20 $\mu$L/well of lysis solution (Promega, Cat. No. G182A) was added to the target cell maximum release group and the lysis solution control group. After 45 min, the cell lysis rates (killing rates) were measured using a CytoTox96® nonradioactive cytotoxicity assay kit (Promega, G1780). Trastuzumab, T-DM1 (trastuzumab-emtansine conjugate, under trade name Kadcyla®), the combination of trastuzumab and pertuzumab (1:1), and Expi HER2-1 were used as the control drugs.

$$\text{Rate of lysis (\%)} = (OD_{\text{administration group}} - OD_{\text{target cell + effector cell group}})/(OD_{\text{target cell maximum release group}} - OD_{\text{target cell group}}) \times 100\%$$

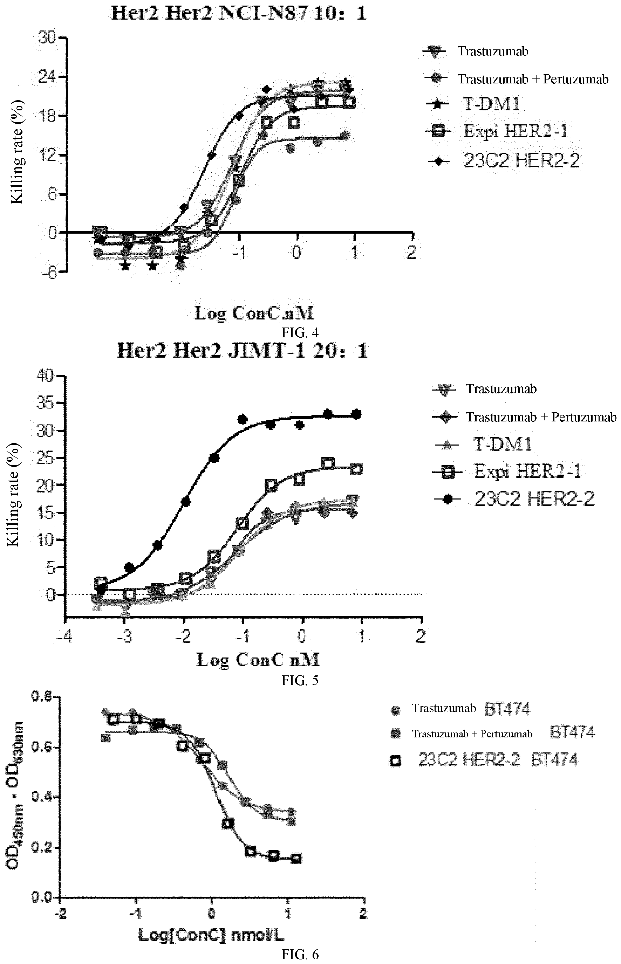

FIG. 4 shows the killing rates of the combination of trastuzumab and pertuzumab, trastuzumab, T-DM1 and the anti-Her2 bispecific antibodies against NCI-N87 tumor cells. The ADCC-enhanced anti-Her2 bispecific antibody 23C2 HER2-2 had a better killing effect on NCI-N87 tumor cells than the combination of trastuzumab and pertuzumab, trastuzumab, T-DM1 and Expi HER2-1, wherein the $EC_{50}$ of the ADCC-enhanced anti-Her2 bispecific antibody 23C2 HER2-2 is 0.02447 nM, the $EC_{50}$ of the combination of trastuzumab and pertuzumab is 0.08267 nM, the $EC_{50}$ of Expi HER2-1 is 0.1048 nM, the $EC_{50}$ of T-DM1 is 0.07392 nM, and the $EC_{50}$ of trastuzumab is 0.07468 nM.

**Example 10. Killing of Trastuzumab-Resistant Cell JIMT-1 by Anti-Her2 Bispecific Antibodies**

The killing effects of the anti-Her2 bispecific antibodies on target cells (JIMT-1, source: AddexBio, Cat. No. C0006005) were studied using NK cells provided by human PBMCs (peripheral blood mononuclear cells; effector cells). $EC_{50}$ was calculated, and the *in vitro* activity of the anti-Her2 bispecific antibodies was assessed.

The specific procedures are as follows:

JIMT-1 cells were adjusted to a cell density of $3 \times 10^5$ cells/mL using 1640 medium containing 2% FBS (fetal bovine serum) and seeded in a 96-well cell culture plate (eppendorf, Cat. No. 0030730199) at 50 $\mu$L per well.

Different concentrations of the anti-Her2 bispecific antibodies or control drugs (8.1 nM, 2.7 nM, 0.9 nM, 0.3 nM, 0.1 nM, 0.03 nM, 0.01 nM, 0.003 nM, 0.001 nM and 0.0004 nM) were prepared using 1640 medium and added to the above 96-well cell culture plate at 50 $\mu$L per well. Human PBMCs were adjusted to a cell density of $1.5 \times 10^6$ cells/mL using 1640 medium and added at 100 $\mu$L per well. An administration group (target cell + effector cell + antibody or control drug), a target cell group (BT474 cell), an effector cell group (human PBMC), a target cell + effector cell group, a blank control group (medium) and a lysis solution control group, and a target cell maximum release group (target cell + lysis solution) were set, with the effector-to-target cell ratio being 20:1. Forty-five minutes prior to the assay, 20 $\mu$L/well of lysis solution (Promega, Cat. No. G182A) was added to the target cell maximum release group and the lysis solution control group. After 45 min, the cell lysis rates (killing rates) were measured using a CytoTox96® nonradioactive cytotoxicity assay kit (Promega, G1780). Trastuzumab, T-DM1, the combination of trastuzumab and pertuzumab (1:1), and Expi HER2-1 were used as the control drugs.

$$\text{Rate of lysis (\%)} = (OD_{\text{administration group}} - OD_{\text{target cell + effector cell group}})/(OD_{\text{target cell maximum release group}} - OD_{\text{target cell group}}) \times 100\%$$

FIG. 5 shows the killing rates of the combination of trastuzumab and pertuzumab, trastuzumab, T-DM1 and the anti-Her2 bispecific antibodies against JIMT-1 tumor cells. The ADCC-enhanced anti-Her2 bispecific antibody 23C2 HER2-2 had a better killing effect on JIMT-1 tumor cells than the combination of trastuzumab and pertuzumab, trastuzumab, T-DM1 and Expi HER2-1, wherein the $EC_{50}$ of the ADCC-enhanced anti-Her2 bispecific antibody 23C2 HER2-2 is 0.01006 nM, the $EC_{50}$ of the combination of trastuzumab and pertuzumab is 0.06727 nM, the $EC_{50}$ of Expi HER2-1 is 0.08066 nM, the $EC_{50}$ of T-DM1 is 0.08357 nM, and the $EC_{50}$ of trastuzumab is 0.07443 nM; in addition, the anti-Her2 bispecific antibody 23C2 HER2-2 showed a higher cell lysis rate.

**Example 11. Inhibition of Proliferation of BT474 Her2+++ Tumor Cells by Anti-Her2 Bispecific Antibodies**

23C2 Her2-2, trastuzumab and pertuzumab were diluted with DMEM/F12 medium (GIBCO, Cat. No. 11330-032) containing 2% FBS (fetal bovine serum, manufacturer: GIBCO, Cat. No. 10099-141) to a final concentration of 3.2 μg/mL, and then serially diluted in a ratio of 1:1 until 9 concentrations (1.6 μg/mL, 0.8 μg/mL, 0.4 μg/mL, 0.2 μg/mL, 0.1 μg/mL, 0.05 μg/mL, 0.025 μg/mL, 0.0125 μg/mL and 0.00625 μg/mL) were obtained. BT474 Her2+++ cells growing at log phase were collected, adjusted to a density of $1 \times 10^5$ cells/mL, and plated at 100 μL per well, and a blank well without any cells was set as a control. The above serially diluted antibodies were added at 50 μL per well. The plate was incubated in an incubator at 37 °C with 5% $CO_2$ for 5 days. The culture medium was discarded, and CCK-8 (Dojindo, Japan, Cat. No. CK04) working solution was added at 100 μL per well. The plate was incubated for 4-5 h for color developing and placed in a microplate reader (manufacturer: Thermo, Model: VarioskanFlash). The absorbance values at a wavelength of 450 nm were read and recorded using a reference wavelength of 630 nm. The proliferation inhibition rates against the tumor cells were calculated.

The results are shown in FIG. 6. The proliferation inhibition rate of the ADCC-enhanced anti-Her2 bispecific antibody 23C2 Her2-2 against BT474 tumor cells is 78.38%, which is better than that of trastuzumab (54.12%) and that of the combination of trastuzumab and pertuzumab (53.7%).

**Example 12. Inhibition of NCI-N87 Her2++ Gastric Cancer Nude Mice Xenograft Tumor by Anti-Her2 Bispecific Antibodies**

The *in vivo* efficacy of the anti-Her2 bispecific antibodies was assessed in a mouse xenograft model using NCI-N87 Her2++ gastric cancer cells (the Cell Bank of Type Culture Collection Committee of the Chinese Academy of Sciences). NCI-N87 Her2++ gastric cancer cells were prepared at a concentration of $5 \times 10^7$ cells/mL and inoculated into nude mice (from Changzhou Cavens Laboratory Animal Ltd., 14-17 g, male, housed in an SPF environment) at the right side armpit at 0.1 mL/mouse. The diameter of the nude mouse xenograft tumor was measured using a vernier caliper, and the animals were randomized into 5 groups when the tumors grew to 100-250 mm³:

Group 1: control
Group 2: Expi Her2-1, 10 mg/kg
Group 3: 23C2 Her2-2, 5 mg/kg
Group 4: 23C2 Her2-2, 10 mg/kg
Group 5: Per + Tra (the combination of trastuzumab and pertuzumab), 5 + 5 mg/kg

Each administration group was intravenously injected with a corresponding dose of the drug twice a week for about 3 consecutive weeks (6 injections). Each group was dosed at 10 mL/kg mouse body weight except that the two drugs in group 5 (the combination group) were each administered at 5 mL/kg mouse body weight. Group 1 was dosed i.v. with PBS (Hyclone; Cat. No. sh30256.01) at 10 mL/kg. In the combination group, trastuzumab was administered at least 30 min after pertuzumab was administered.

The anti-tumor effects of the test drugs were dynamically monitored by measuring the tumor volume. The tumor volume was measured 2-3 times a week (the specific time points of measurement are shown in Table 8), and meanwhile the mice were weighed; the data were recorded. The general behavior of the mice was observed every day.

Detection index:

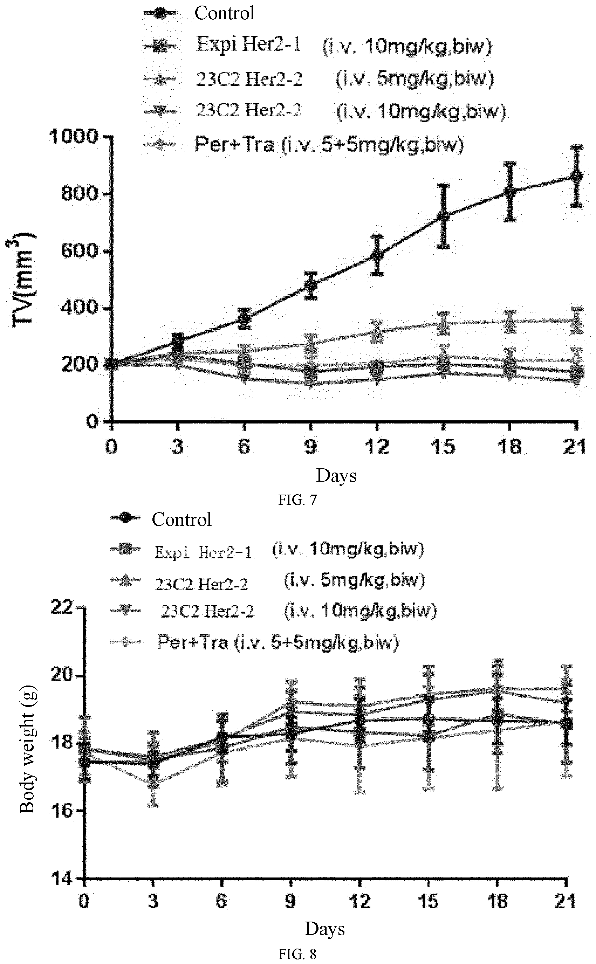

Tumor volume (TV), calculated as follows: TV = $1/2 \times a \times b^2$, where a and b represent length and width, respectively. In this experiment, administration was started on d0 and performed 6 times (on d0, d3, d7, d10, d14 and d17). By d21 of the experiment, no animals died. The mean body weight of the mice in each group showed an upward trend (FIG. 8). The drugs had no significant toxic effect.

By d21, the effects of group 2 (10 mg/kg), group 3 (5 mg/kg), group 4 (10 mg/kg) and group 5 (5 + 5 mg/kg) on NCI-N87 gastric cancer nude mouse xenograft tumor volume are shown in Table 7 and FIG. 7. 23C2 Her2-2, at the dose of 10 mg/kg, had a better inhibitory effect on the NCI-N87 gastric cancer nude mouse xenograft tumor than Expi Her2-1 (10 mg/kg) and the Per + Tra combination group (5 + 5 mg/kg).

Table 8: Effects of anti-HER2 bispecific antibodies on NCI-N87 gastric cancer nude mouse xenograft tumor volume (mean ± SD)

| Group | Dose (mg/kg) | Route of administration | Number of animals (mice) | | TV (mm³) | | | | | | | |
|---|---|---|---|---|---|---|---|---|---|---|---|---|
| | | | d0 | d21 | d0 | d3 | d6 | d9 | d12 | d15 | d18 | d21 |
| Control | - | i.v. | 6 | 6 | 204±40 | 284±58 | 363±78 | 481±109 | 586±161 | 724±261 | 808±241 | 863±251 |
| Expi Her2-1 | 10 | i.v. | 6 | 6 | 204±23 | 235±46 | 207±35 | 179±30 | 195±24 | 204±44 | 195±50 | 178±35 |
| 23C2 Her2-2 | 5 | i.v. | 6 | 6 | 204±31 | 246±37 | 250±47 | 277±67 | 318±79 | 348±86 | 353±84 | 358±101 |
| 23C2 Her2-2 | 10 | i.v. | 6 | 6 | 204±44 | 201±39 | 154±39 | 136±33 | 151±35 | 173±46 | 165±35 | 144±29 |
| Per+Tra | 5+5 | i.v. | 6 | 6 | 204±34 | 224±48 | 199±59 | 202±65 | 205±45 | 231±93 | 218±94 | 218±91 |

Note: Administration was performed on d0, d3, d7, d10, d14 and d17, making a total of 6 administrations.

**Claims**

1. An antigen-binding construct, comprising a first antigen-binding fragment that is monovalent and specifically binds to ECD4 antigen of HER2 on an HER2-expressing cell, wherein the first antigen-binding fragment is an scFv; a heavy chain variable region of the first antigen-binding fragment comprises an amino acid mutation at position 30 according to the Kabat numbering system, and preferably, the amino acid is mutated into E; or/and a light chain variable region of the first antigen-binding fragment comprises an amino acid mutation at position 53 according to the Kabat numbering system, and preferably, the amino acid is mutated into Y

2. The antigen-binding construct according to claim 1, wherein the heavy chain variable region of the first antigen-binding fragment comprises a K30E mutation according to the Kabat numbering system, or/and the light chain variable region of the first antigen-binding fragment comprises an F53Y mutation according to the Kabat numbering system.

3. The antigen-binding construct according to claim 1 or 2, wherein the antigen-binding construct is an antibody.

4. The antigen-binding construct according to any one of claims 1-3, wherein the first antigen-binding fragment comprises a group of CDRs selected from:

   i. a group comprising a heavy chain CDR1, a heavy chain CDR2 and a heavy chain CDR3, wherein the heavy chain CDR1, the heavy chain CDR2 and the heavy chain CDR3 comprise the amino acid sequences according to SEQ ID NOs: 27, 28 and 29, respectively;
   ii. a group comprising a heavy chain CDR1, a heavy chain CDR2 and a heavy chain CDR3, wherein the heavy chain CDR1, the heavy chain CDR2 and the heavy chain CDR3 comprise amino acid sequences having at least 80% identity to the amino acid sequences according to SEQ ID NOs: 27, 28 and 29, respectively;
   iii. a group comprising a light chain CDR1, a light chain CDR2 and a light chain CDR3, wherein the light chain CDR1, the light chain CDR2 and the light chain CDR3 comprise the amino acid sequences according to SEQ ID NOs: 30, 34 and 32, respectively;
   iv. a group comprising a light chain CDR1, a light chain CDR2 and a light chain CDR3, wherein the light chain CDR1, the light chain CDR2 and the light chain CDR3 comprise amino acid sequences having at least 80% identity to the amino acid sequences according to SEQ ID NOs: 30, 34 and 32, respectively;
   v. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 27, 28 and 29, respectively; and the light chain CDR1, the light chain CDR2 and the light chain CDR3 comprise the amino acid sequences according to SEQ ID NOs: 30, 34 and 32, respectively;
   vi. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise amino acid sequences having at least 80% identity to the amino acid sequences according to SEQ ID NOs: 27, 28 and 29, respectively; and the light chain CDR1, the light chain CDR2 and the light chain CDR3 comprise amino acid sequences having at least 80% identity to the amino acid sequences according to SEQ ID NOs: 30, 34 and 32, respectively;
   vii. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 43, 28 and 29, respectively; and the light chain CDR1, the light chain CDR2 and the light chain CDR3 comprise the amino acid sequences according to SEQ ID NOs: 30, 31 and 32, respectively;
   viii. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 43, 28 and 29, respectively; and the light chain CDR1, the light chain CDR2 and the light chain CDR3 comprise the amino acid sequences according to SEQ ID NOs: 30, 33 and 32, respectively; and
   ix. a group comprising a heavy chain CDR1, a heavy chain CDR2, a heavy chain CDR3, a light chain CDR1, a light chain CDR2 and a light chain CDR3, wherein the heavy chain CDR1, the heavy chain CDR2 and the heavy chain CDR3 comprise the amino acid sequences according to SEQ ID NOs: 44, 28 and 29, respectively; and the light chain CDR1, the light chain CDR2 and the light chain CDR3 comprise the amino acid sequences according to SEQ ID NOs: 30, 31 and 32, respectively.

5. The antigen-binding construct according to any one of claims 1-4, wherein the first antigen-binding fragment comprises a heavy chain variable region of trastuzumab or a mutant thereof, and a light chain variable region of trastuzumab or a mutant thereof; the mutant of the heavy chain variable region comprises a K30E mutation according to the Kabat numbering system, or/and the mutant of the light chain variable region comprises an F53Y mutation according to the Kabat numbering system.

6. The antigen-binding construct according to any one of claims 4-5, wherein the first antigen-binding fragment is selected from:

   i. the first antigen-binding fragment comprising a heavy chain variable region and a light chain variable region, which comprise the amino acid sequences according to SEQ ID NOs: 41 and 42, respectively;
   ii. the first antigen-binding fragment comprising a heavy chain variable region and a light chain variable region, which comprise amino acid sequences having at least 80% identity to the amino acid sequences according to SEQ ID NOs: 41 and 42, respectively;
   iii. the first antigen-binding fragment comprising a heavy chain variable region and a light chain variable region, which comprise the amino acid sequences according to SEQ ID NOs: 35 and 36, respectively;
   iv. the first antigen-binding fragment comprising a heavy chain variable region and a light chain variable region, which comprise the amino acid sequences according to SEQ ID NOs: 35 and 39, respectively; and
   v. the first antigen-binding fragment comprising a heavy chain variable region and a light chain variable region, which comprise the amino acid sequences according to SEQ ID NOs: 40 and 36, respectively.

7. The antigen-binding construct according to any one of claims 1-6, wherein a VH and a VL of the first antigen-binding fragment are arranged from N-terminus to C-terminus in the following order: VH-linker-VL.

8. The antigen-binding construct according to any one of claims 1-7, wherein the antigen-binding construct further comprises a second antigen-binding fragment that is monovalent and specifically binds to ECD2 antigen of HER2 on an HER2-expressing cell.

9. The antigen-binding construct according to claim 8, wherein the second antigen-binding fragment is an Fab.

10. The antigen-binding construct according to claim 8 or 9, wherein the second antigen-binding fragment comprises a heavy chain CDR1, a heavy chain CDR2, a heavy chain CDR3, a light chain CDR1, a light chain CDR2 and a light chain CDR3 of pertuzumab.

11. The antigen-binding construct according to claim 8 or 9, wherein the second antigen-binding fragment comprises a heavy chain variable region of pertuzumab and a light chain variable region of pertuzumab.

12. The antigen-binding construct according to any one of claims 8-11, wherein the antigen-binding construct comprises an immunoglobulin functional domain operably linked to the first antigen-binding fragment or/and the second antigen-binding fragment, the immunoglobulin functional domain comprising: i. one or more of a CL, a CH1, a CH2 and a CH3; or ii. an Fc.

13. The antigen-binding construct according to claim 12, wherein the CL, CH1, CH2, CH3 and Fc are derived from CL, CH1, CH2, CH3 and Fc of human IgG, respectively.

14. The antigen-binding construct according to claim 12, wherein the CL, CH1, CH2, CH3 or Fc has a modification or does not have a modification; preferably, the modification the CH3 or Fc has is, e.g., an amino acid substitution at position 435 or/and position 436 according to the Kabat numbering system.

15. The antigen-binding construct according to claim 12, wherein the Fc is a dimeric Fc comprising a first Fc polypeptide and a second Fc polypeptide; the first antigen-binding fragment is operably linked to the first Fc polypeptide, and the second antigen-binding fragment is operably linked to the second Fc polypeptide.

16. The antigen-binding construct according to any one of claims 1-15, wherein the antigen-binding construct is a bispecific antibody or a multispecific antibody.

17. The antigen-binding construct according to any one of claims 1-15, wherein the antigen-binding construct is bivalent or polyvalent.

**18.** The antigen-binding construct according to any one of claims 1-7, wherein the antigen-binding construct is a monospecific antibody; wherein the antigen-binding construct is monovalent, bivalent or polyvalent.

**19.** The antigen-binding construct according to claim 1, wherein the antigen-binding construct is selected from the following group:

   i. the antigen-binding construct is a bivalent bispecific antibody, comprising: a heavy chain comprising SEQ ID NO: 11, a heavy chain comprising SEQ ID NO: 13, and a light chain comprising SEQ ID NO: 15;
   ii. the antigen-binding construct is a bivalent bispecific antibody, comprising: a heavy chain comprising SEQ ID NO: 21, a heavy chain comprising SEQ ID NO: 13, and a light chain comprising SEQ ID NO: 15;
   iii. the antigen-binding construct is a bivalent bispecific antibody, comprising: a heavy chain comprising SEQ ID NO: 23, a heavy chain comprising SEQ ID NO: 13, and a light chain comprising SEQ ID NO: 15;
   iv. the antigen-binding construct is a bivalent monospecific antibody, comprising the amino acid sequence of SEQ ID NO: 3;
   v. the antigen-binding construct is a bivalent monospecific antibody, comprising the amino acid sequence of SEQ ID NO: 9; and
   vi. the antigen-binding construct is a bivalent monospecific antibody, comprising the amino acid sequence of SEQ ID NO: 51.

**20.** The antigen-binding construct according to any one of claims 1-19, wherein the antigen-binding construct has reduced aggregation compared to antigen-binding constructs in which the amino acid at position 30 is not E or/and the amino acid at position 53 is not Y; or/and the antigen-binding construct does not have significantly reduced binding affinity for ECD4 antigen or/and ECD2 antigen of HER2 compared to antigen-binding constructs in which the amino acid at position 30 is not E or/and the amino acid at position 53 is not Y

**21.** The antigen-binding construct according to any one of claims 1-20, wherein the antigen-binding construct is afucosylated.

**22.** A pharmaceutical composition, comprising the antigen-binding construct according to any one of claims 1-21 and a pharmaceutically acceptable carrier.

**23.** An isolated nucleic acid or a collection of isolated nucleic acids, comprising at least one nucleic acid sequence encoding at least one antigen-binding fragment of the antigen-binding construct according to any one of claims 1-21.

**24.** A vector or a collection of vectors, comprising one or more of the nucleic acid and the collection of nucleic acids according to claim 23.

**25.** An isolated cell, comprising the nucleic acid or the collection of nucleic acids according to claim 23, or the vector or the collection of vectors according to claim 24.

**26.** A method for preparing the antigen-binding construct according to any one of claims 1-21, comprising: culturing a host cell under conditions suitable for expression of the antigen-binding construct, wherein the host cell comprises a nucleic acid encoding the antigen-binding construct, or the vector or the collection of vectors according to claim 24; and purifying the construct.

**27.** A method for treating a subject having an HER2-expressing tumor, comprising administering to the subject a therapeutically effective amount of the antigen-binding construct according to any one of claims 1-21 or the pharmaceutical composition according to claim 22.

**28.** The method according to claim 27, comprising contacting a tumor cell with the antigen-binding construct so that an HER2-expressing tumor cell is killed or growth of an HER2-expressing tumor cell is inhibited.

**29.** The method according to claim 27 or 28, wherein the tumor is biliary tract cancer, carcinosarcoma, esophageal cancer, gastroesophageal junction cancer, breast cancer, gastric cancer, pancreatic cancer, head and neck cancer, colorectal cancer, renal cancer, cervical cancer, ovarian cancer, endometrial cancer, uterine cancer, malignant melanoma, pharyngeal cancer, oral cancer, or skin cancer.

**30.** The method according to any one of claims 27-29, comprising administering to a cell an effective amount of the antigen-binding construct or the pharmaceutical composition so that HER2 signaling in the cell is inhibited, reduced or blocked.

31. A method for enhancing resistance of an antigen-binding construct to aggregation, wherein the antigen-binding construct comprises a first antigen-binding fragment that is monovalent and specifically binds to ECD4 antigen of HER2 on an HER2-expressing cell, the first antigen-binding fragment being an scFv; the method comprises modifying the antigen-binding construct so that a heavy chain variable region of the first antigen-binding fragment comprises an amino acid mutation at position 30 according to the Kabat numbering system and the amino acid is mutated into E, or/and that a light chain variable region of the first antigen-binding fragment comprises an amino acid mutation at position 53 according to the Kabat numbering system and the amino acid is mutated into Y.

FIG. 1

FIG. 2

FIG. 3

### Her2 Her2 NCI-N87 10：1

FIG. 4

### Her2 Her2 JIMT-1 20：1

FIG. 5

FIG. 6

Control
Expi Her2-1    (i.v. 10mg/kg,biw)
23C2 Her2-2     (i.v. 5mg/kg,biw)
23C2 Her2-2     (i.v. 10mg/kg,biw)
Per+Tra (i.v. 5+5mg/kg,biw)

FIG. 7

Control
Expi Her2-1    (i.v. 10mg/kg,biw)
23C2 Her2-2     (i.v. 5mg/kg,biw)
23C2 Her2-2     (i.v. 10mg/kg,biw)
Per+Tra (i.v. 5+5mg/kg,biw)

FIG. 8

FIG. 9

## INTERNATIONAL SEARCH REPORT

| | International application No. |
|---|---|
| | **PCT/CN2021/090794** |

### A. CLASSIFICATION OF SUBJECT MATTER

C07K 16/28(2006.01)i; C12N 15/13(2006.01)i; C12N 15/63(2006.01)i; C12N 5/16(2006.01)i; A61K 39/395(2006.01)i; A61P 35/00(2006.01)i

According to International Patent Classification (IPC) or to both national classification and IPC

### B. FIELDS SEARCHED

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS; CNMED; CNTXT; DWPI; SIPOABS; EPTXT; WOTXT; USTXT; JPTXT; CNKI; PUBMED; ISI Web of Science and Keywords: 抗体, 人表皮生长因子受体2, 乳腺癌, 癌, 肿瘤, 抗原, 突变, K30E, K30, F53Y, F53, antibody, human epidermal growth factor receptor 2, Her2, human epidermal growth factor receptor 2, ErbB2, EGFR, IHC, mutaition, cancer, tumour, antigen etc.。 中国专利生物序列检索系统, Chinese Patent Biological Sequence Retrieval System; Genbank和检索的序列, Genbank and search sequence: SEQ ID NOs: 1-55。

### C. DOCUMENTS CONSIDERED TO BE RELEVANT

| Category* | Citation of document, with indication, where appropriate, of the relevant passages | Relevant to claim No. |
|---|---|---|
| A | CN 105980409 A (ZYMEWORKS INC.) 28 September 2016 (2016-09-28) see entire document, especially claims 1-77 | 1-31 |
| A | CN 105940113 A (ZYMEWORKS INC.) 14 September 2016 (2016-09-14) see entire document, especially claims 1-112 | 1-31 |
| A | CN 109715671 A (BEIJING MABWORKS BIOTECH CO., LTD.) 03 May 2019 (2019-05-03) see entire document, especially claims 1-20 | 1-31 |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **17 July 2021** | **02 August 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2021/090794** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**

| International application No. |
| --- |
| **PCT/CN2021/090794** |

| **Box No. II**     **Observations where certain claims were found unsearchable (Continuation of item 2 of first sheet)** |
| --- |

This international search report has not been established in respect of certain claims under Article 17(2)(a) for the following reasons:

1.  ☑  Claims Nos.: **27-30**
        because they relate to subject matter not required to be searched by this Authority, namely:

   [1]  PCT Rule 39.1(iv) - methods for treatment of the human or animal body by surgery or therapy. The
        reasonably anticipated subject matter is the application of the antigen-binding construct of any one
        of claims 1-21 or the pharmaceutical composition of claim 22 in the preparation of a drug for the
        treatment of a subject suffering from a HER2 expressing tumor.

2.  ☐  Claims Nos.:
        because they relate to parts of the international application that do not comply with the prescribed requirements to such an
        extent that no meaningful international search can be carried out, specifically:

3.  ☐  Claims Nos.:
        because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

International application No.

**PCT/CN2021/090794**

| Patent document cited in search report | | | Publication date (day/month/year) | Patent family member(s) | | | Publication date (day/month/year) |
|---|---|---|---|---|---|---|---|
| CN | 105980409 | A | 28 September 2016 | US | 10947319 | B2 | 16 March 2021 |
| | | | | KR | 20160091961 | A | 03 August 2016 |
| | | | | EP | 3074424 | A1 | 05 October 2016 |
| | | | | AU | 2014357292 | B2 | 25 June 2020 |
| | | | | US | 2018179295 | A1 | 28 June 2018 |
| | | | | US | 2016289335 | A1 | 06 October 2016 |
| | | | | US | 10000576 | B1 | 19 June 2018 |
| | | | | EP | 3074424 | A4 | 14 June 2017 |
| | | | | WO | 2015077891 | A1 | 04 June 2015 |
| | | | | MX | 2016006572 | A | 09 December 2016 |
| | | | | AU | 2020239643 | A1 | 15 October 2020 |
| | | | | JP | 6817064 | B2 | 20 January 2021 |
| | | | | AU | 2014357292 | A1 | 23 June 2016 |
| | | | | JP | 2019205432 | A | 05 December 2019 |
| | | | | BR | 112016012157 | A2 | 22 May 2018 |
| | | | | RU | 2016125551 | A | 09 January 2018 |
| | | | | US | 2018282429 | A1 | 04 October 2018 |
| | | | | CA | 2931356 | A1 | 04 June 2015 |
| | | | | JP | 6727379 | B2 | 22 July 2020 |
| | | | | JP | 2017503480 | A | 02 February 2017 |
| | | | | RU | 2737882 | C2 | 04 December 2020 |
| CN | 105940113 | A | 14 September 2016 | WO | 2015073721 | A1 | 21 May 2015 |
| | | | | KR | 20160083949 | A | 12 July 2016 |
| | | | | CA | 2930307 | A1 | 21 May 2015 |
| | | | | AU | 2014348552 | A1 | 02 June 2016 |
| | | | | EP | 3068892 | A1 | 21 September 2016 |
| | | | | US | 10273303 | B2 | 30 April 2019 |
| | | | | EP | 3068892 | A4 | 31 May 2017 |
| | | | | MX | 2016006301 | A | 16 December 2016 |
| | | | | US | 2016289328 | A1 | 06 October 2016 |
| | | | | JP | 2016538283 | A | 08 December 2016 |
| CN | 109715671 | A | 03 May 2019 | EP | 3487888 | A4 | 01 April 2020 |
| | | | | CN | 109715671 | B | 21 August 2020 |
| | | | | JP | 2019528779 | A | 17 October 2019 |
| | | | | WO | 2018014864 | A1 | 25 January 2018 |
| | | | | CA | 3031330 | C | 28 April 2020 |
| | | | | AU | 2017298251 | B2 | 06 February 2020 |
| | | | | CN | 107446045 | A | 08 December 2017 |
| | | | | US | 9745382 | B1 | 29 August 2017 |
| | | | | EP | 3487888 | A1 | 29 May 2019 |
| | | | | AU | 2017298251 | A1 | 14 February 2019 |
| | | | | CA | 3031330 | A1 | 25 January 2018 |
| | | | | JP | 6823175 | B2 | 27 January 2021 |

Form PCT/ISA/210 (patent family annex) (January 2015)

## REFERENCES CITED IN THE DESCRIPTION

**Patent documents cited in the description**

- WO 2019195535 A

- US 5945311 A

**Non-patent literature cited in the description**

- **E. MEYERS ; W. MILLER.** *Comput. Appl. Biosci.,* 1988, vol. 4, 11-17
- **NEEDLEMAN ; WUNSCH.** *J. Mol. Biol.,* 1970, 484-453
- **T. E. CREIGHTON.** Proteins: Structures and Molecular Properties. W. H. Freeman and company, 1993
- **A. L. LEHNINGER.** Biochemistry. Worth Publishers, Inc,

- **SAMBROOK et al.** Molecular Cloning: A Laboratory Manual. 1989
- Methods In Enzymology. Academic Press, Inc,
- Remington's PharmaceuticalSciences. Mack Publishing Company, 1990
- Carey and Sundberg Advanced Organic Chemistry. Plenum Press, 1992, vol. A,B